# EUROPEAN PATENT SPECIFICATION

(11) **EP 1 901 672 B1**
(45) Date of publication and mention of the grant of the patent: **30.12.2009**
(21) Application number: 06770875.0
(22) Date of filing: 19.05.2006
(51) Int. Cl.: A61B 18/12, G06F 19/00

(54) **USER INTERFACE FOR A PORTABLE THERAPY DELIVERY DEVICE**
ANWENDERSCHNITTSTELLE FÜR EINE TRAGBARE THERAPEUTISCHE VORRICHTUNG
INTERFACE UTILISATEUR POUR UN DISPOSITIF PORTABLE D'ADMINISTRATION D'UNE THERAPIE

(30) Priority: 20.05.2005 US 682936 P; 28.04.2006 US 414623; 28.04.2006 US 414501; 28.04.2006 US 414503; 28.04.2006 US 414613
(43) Date of publication of application: 26.03.2008
(73) Proprietor: Medtronic, Inc., Minneapolis, MN 55432-5604 (US)
(72) Inventor: CHRISTOPHERSON, Mark, A., Shoreview, MN 55126 (US); SKWAREK, Thomas, R., Shoreview, MN 55126 (US)
(74) Representative: Hughes, Andrea Michelle
(86) International application number: PCT/US2006/019782
(87) International publication number: WO 2006/127629

(56) References cited:
- WO-A-00/66015
- US-A- 5 345 362
- US-A- 5 871 481
- US-A1- 5 582 588
- US-A1- 2002 058 933
- US-A1- 2003 093 503
- US-A1- 2004 082 946
- US-B1- 6 280 440

## Description

### TECHNICAL FIELD

The invention relates to medical devices and, more particularly, to devices for controlling therapy delivery.

### BACKGROUND

While some patients must undergo major surgery to treat a diagnosed problem, other therapies may be performed quickly and at a variety of locations. Some of these therapies may include tissue ablation, tissue removal, cauterization, ultrasound therapy, and implantable device programming. Performing some therapies without an operating room enables more patient treatments at a lower cost. These outpatient routines are becoming increasingly popular with both physicians and patients.

Since outpatient procedures are commonly performed in small clinics with limited space for large therapy systems, portable therapy devices allow small clinics to treat patients with a limited number of operating or procedure rooms. Alternatively, some portable devices are moved to the patient's room and the procedure is performed in that room. These portable devices may have wheels to roll the device between rooms or be light enough for a user to carry between locations. Each procedure may be performed by a physician and may require one or more assistants.

One example of an outpatient therapy is treatment for benign prostatic hyperplasia (BPH). BPH is a condition caused by the second period of continued prostate gland growth. This growth begins after a man is approximately 25 years old and may begin to cause health problems after 40 years of age. The prostate growth eventually begins to constrict the urethra and may cause problems with urination and bladder functionality. While invasive surgery can remove the enlarged prostate, minimally invasive surgery has recently become an effective alternative. This therapy introduces a catheter and needle into the urethra and to the prostate. The needle is entered into the prostate where it heats and destroys a portion of the surrounding prostate tissue. In this example, the patient may enjoy effective therapy without any major side effects, and the physician may perform a less invasive procedure that incorporates less risk with respect to invasive surgery. A system comprising the features of the preamble of claim 1 is known from US-A-2004/0082946.

### SUMMARY

This disclosure is directed to a system as defined in claim 1 or a device as defined in claim 13, that may be used to deliver a plurality of therapies through the use of one portable system. The system includes a pop-up interactive display that is a touch screen for controlling the system while delivering therapy through the use of the delivery device. The touch screen is used by a physician to control the therapy or communicate data to or from another device. In addition, the therapy delivery device may include an operation indicator, a signal generator, a connector board port, a connector board that removably couples to the connector board port, and a fluid pump. Generated signals may be used by a peripheral accessory connected to the generator through the connector board coupled to the connector board port. In particular, the generator may generate radio frequency (RF) energy for the purpose of prostate tissue ablation.

Portable therapy devices are increasingly important and valuable to medical clinics because they allow patients to be treated in any area or room of the patient. This portability may lessen the cost of therapy and enable a clinic to perform a wider variety of therapies than with larger systems. In addition, treatment efficacy increases when these portable therapy devices employ simple and easy to use controls that lessen complexity, and possibly error rate, of the procedure.

Integrating a touch screen user interface into a portable therapy device may allow a physician to easily navigate the software of the device without having to press small buttons or manipulate other controls of a keyboard or joystick. In addition, the physician only has to look at the screen, instead of constantly looking between the input device and the screen. The physician may use the touch screen with a gloved finger, which allows the physician to use the touch screen during therapy and the flat surface may facilitate easy cleaning and sterilization after the procedure.

In an exemplary use of the portable therapy device, the generator may generate radio frequency (RF) energy for the purpose of prostate tissue ablation. The energy may be directed through a connected lead of an ablation device to an electrode or electrodes placed at a certain location within the prostate. In addition, the system may provide fluid to cool the urethra and fluid to flow from an electrode during ablation to increase the efficacy of treatment.

Not only would the device be capable of modification to treat other conditions, the device may be conducive for equipment upgrades as technology or treatment methods advance. For example, an endoscopic camera may be implemented at the tip of the ablation catheter to help the physician guide electrodes into place and monitor treated tissue.

Although the device described herein may be especially applicable to an RF generator device and prostate tissue ablation, alternative diagnostic and therapeutic procedures may be used in the clinic with this device. Exemplary diagnostic procedures may include general endoscopy, gastric endoscopy, ultrasound imaging, blood pressure measurements, and blood oxygenation measurements. Alternative therapies may include ultrasound treatments, cauterizing, and implanted device programming.

In various embodiments, the device described in this disclosure may provide one or more advantages. For example, a touch screen may allow for simple user control without multiple buttons or keys to push. The touch screen may allow for gloved use and easy disinfection. In addition, the touch screen folds down and latches so that the screen is protected by a screen housing when the device is not being used. The light bar at the top of the screen housing may provide valuable operational information when the physician is not in front of the touch screen.

In some cases, the system may have the ability to transfer data between other devices. This aspect may be useful for analyzing therapy data, monitoring patient trends, troubleshooting device problems, and downloading software upgrades. The device may also be able to transfer data to a physician's hand held computer via a USB flash memory device or wireless communications.

The details of one or more embodiments of the invention are set forth in the accompanying drawings and the description below. Other features, objects, and advantages of the invention will be apparent from the description and drawings, and from the claims.

### BRIEF DESCRIPTION OF THE DRAWINGS

FIG. 1 is a conceptual diagram illustrating an example generator system in conjunction with a patient.

FIG. 2 is a top view of an example generator system in the screen closed configuration.

FIG. 3 is a side view of an example generator system in the screen closed configuration.

FIG. 4 is a front view of an example generator system in the screen open configuration.

FIG. 5 is a top view of a peristaltic fluid pump in an open pump bay of an example generator system.

FIG. 6 is a top view of an internal gear fluid pump in an open pump bay of an example generator system.

FIG. 7A is an enlarged side view of an example light bar of an example generator system.

FIGS. 7B and 7C are enlarged end views of two example light bars with slightly different shapes.

FIG. 8A is an enlarged side view of an example removable connector board.

FIG. 8B is a top view of an example removable connector board.

FIG. 9 is functional block diagram illustrating components of an exemplary generator system.

FIG. 10 is a flow diagram illustrating an example technique for operating the generator system in attaching a peripheral accessory and providing therapy to a patient.

FIG. 11 is a flow diagram illustrating an example technique for identifying a connected peripheral accessory and determining its status before providing therapy to a patient.

FIG. 12 is an exemplary screen shot of the main menu provided by the user interface.

FIG. 13 is an exemplary screen shot of the delivery screen when the system becomes operational.

FIG. 14 is an exemplary screen shot of the delivery screen when ablation ' therapy is being delivered.

FIG. 15 is an exemplary screen shot of the delivery screen and a temperature warning message during therapy.

FIG. 16 is an exemplary screen shot of the delivery screen displaying an error message when the therapy is terminated due to the return electrode malfunction.

FIG. 17 is an exemplary screen shot of the delivery screen when the therapy is completed.

FIG. 18 is an exemplary screen shot of the post session menu.

### DETAILED DESCRIPTION

This disclosure is directed to a portable therapy delivery device, or system, to be used by a physician to treat a variety of patient conditions. The portable delivery device may provide a platform for a plurality of peripheral accessories, i.e., therapy or diagnostic devices, to be connected. A platform such as this may be useful to the medical community by offering flexibility in a device that may be used for a variety of purposes. Additionally, costs for the manufacturer, physician, and patient may be decreased by utilizing a platform device which may be slightly modified to perform a number of diagnostic or therapeutic tasks. This platform device may even be used across multiple medical disciplines. Such examples may include any type of tissue ablation (e.g. prostate, heart, liver, mouth, throat, eye, etc.), ultrasound imaging, endoscopy, implantable programming, or any combination of these and other procedures.

For exemplary purposes, the description provided herein is aimed at a portable therapy delivery device that includes hardware and software capable of providing RF ablation to the prostate, in this case using a wet electrode. In some cases, RF ablation may be conducted with a dry electrode. The delivery device provides an RF generator, a fluid pump, a ablation device, and a user interface to control the aspects of the therapy. A needle is introduced to the prostate via the urethra, where it delivers the RF energy to the prostate to ablate surrounding tissue. The circulation of fluid from and/or around the electrode may allow for a greater volume of tissue to be destroyed in a shorter period of time, effectively increasing therapy efficacy. This therapy may also be coupled with other associated therapies or diagnostic equipment attached to the portable therapy delivery device. For example, multiple fluid pumps may be included within the platform or added via USB port control. Additional pumps may enable tissue irrigation for clearing ablated tissue or cooling surrounding tissue.

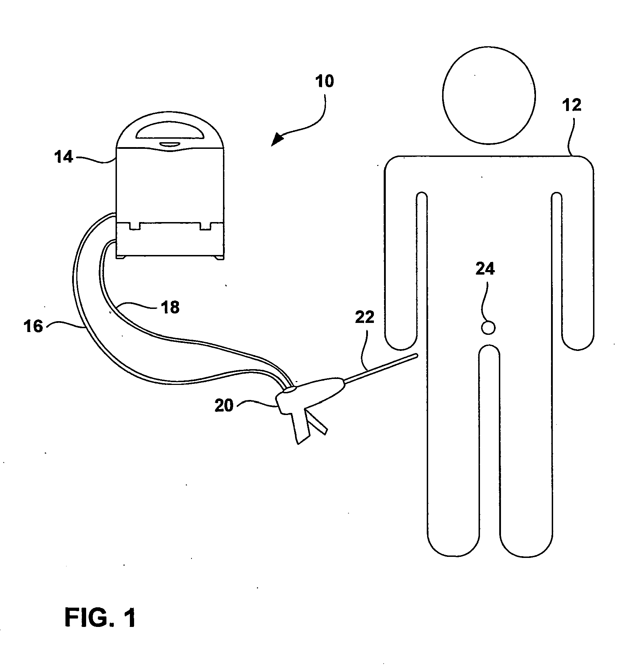

FIG. 1 is a conceptual diagram illustrating an example system 10 in conjunction with a patient 12. As shown in FIG. 1, system 10 may include a portable therapy delivery device (PTD) 14 that delivers therapy to treat a condition of patient 12. In this exemplary embodiment, PTD 14 is a radio frequency (RF) generator that provides RF energy to heat tissue of the prostate gland 24. This ablation of prostate tissue destroys a portion of the enlarged prostate caused by, for example, benign prostatic hyperplasia (BPH). The RF energy is transmitted through electrical cable 16 to ablation device 20. The energy is then transmitted through a probe 22 and is delivered to prostate 24 by an electrode (not shown). In addition to the electrode, a fluid may be pumped out of delivery device 14, through tube 18, into ablation device 20, and through probe 22 to interact with the RF energy being delivered by the electrode. This wet electrode may increase the effective heating area of the electrode and increase therapy efficacy.

In the illustrated example, PTD 14 includes an RF generator that includes circuitry for developing RF energy from an included rechargeable battery or a common electrical outlet. The RF energy is produced within parameters adjusted to provide appropriate prostate tissue heating. The RF current is conveyed from the PTD 14 via an electrical cable 16 which is connected to a connector board of PTD 14. A connector board may be inserted into PTD 14 for this therapy, and it may be replaced with a different connector board for additional therapies or diagnostics. Fluid is provided to the electrode by a pump (not shown) also located within PTD 14. The pump may also be replaceable to enable substitute pumps to be used in this or other therapies.

Therapy energy and other associated functions such as fluid flow are controlled via a graphic user interface located on a color liquid crystal display (LCD), or equivalent screen. The screen may provide images created by the therapy software, and the user may interact with the software by touching the screen at certain, locations indicated by the user interface. In this, embodiment, no additional devices, such as a keyboard or pointer device, are needed to interact with the device. The touch screen may also enable device operation. In some embodiments, the device may require an access code or biometric authorization to use the device. Requiring the physician to provide a fingerprint, for example, may limit unauthorized use of the system.

Connected to PTD 14 are one cable 16 and one tube 18. Cable 16 conveys RF energy and tube 18 conducts fluid from PTD 14 to ablation device 20. Ablation device 20 may be embodied as a hand-held device as shown in FIG. 1. Ablation device 20 may include a trigger to control the start and stop of therapy. The trigger may be pressure sensitive, where increased pressure of the trigger provides an increased amount of RF energy or increase the fluid flow to the tissue of prostate 24. Attached to the distal end of ablation device 20 is a probe 22. The probe may provide a conduit for the fluid and provide isolation between one or more needles that conduct RF energy and patient 12. Since the probe 22 would be entering patient 12 through the urethra, the probe may be very thin in diameter and long enough to reach the prostate in any patient.

Probe 22 may contain one or more electrodes for delivering RF current to the tissue of enlarged prostate 24. Probe 22 may contain one or more needles, each with an electrode, for penetrating into two opposite areas of prostate 24 from the urethra. When RF energy is being delivered, tissue may increase in temperature, which may destroy tissue. This heating may last a few seconds or a few minutes, depending on the condition of prostate 24. In some embodiments, the fluid may exit small holes in the needles and flow around the electrodes. This conducting fluid, e.g., saline, may increase the effective heating area and decrease the heating time. Additionally, ablating tissue in this manner may enable the physician to complete therapy without repositioning the needle.

In some cases, ablation devices may only be used for one patient. Reuse may cause infection and contamination, so it may be desirable for the ablation device to only be used once. A feature on the ablation device may be a smart chip in communication with the PTD 14. For example, when the ablation device is connected to PTD 14, the PTD may request use information from the ablation device. If the device has been used before, the PTD may disable all functions of the ablation device to prevent reuse of the device. Once an ablation device has been used, the smart chip may create a use log to identify the therapy delivered and record that the device has been used. The log may include data of RF energy delivered to the patient, total RF energy delivered in terms of joules or time duration, error messages created, or any other pertinent information.

In some embodiments, additional peripheral accessories, i.e., therapy devices or diagnostic devices, may be available to the physician at one time. For example, the ablation device for ablating prostate tissue might be coupled with an endoscopic camera for locating the prostate and monitoring therapy. The camera images may then be transferred back to PTD 14 and presented on the screen in real-time. Other examples may include ultrasound imaging coupled with ablation therapy or programming implanted medical devices. The flexible platform of the PTD 14 may allow various diagnostic and therapy combinations to be combined into one device.

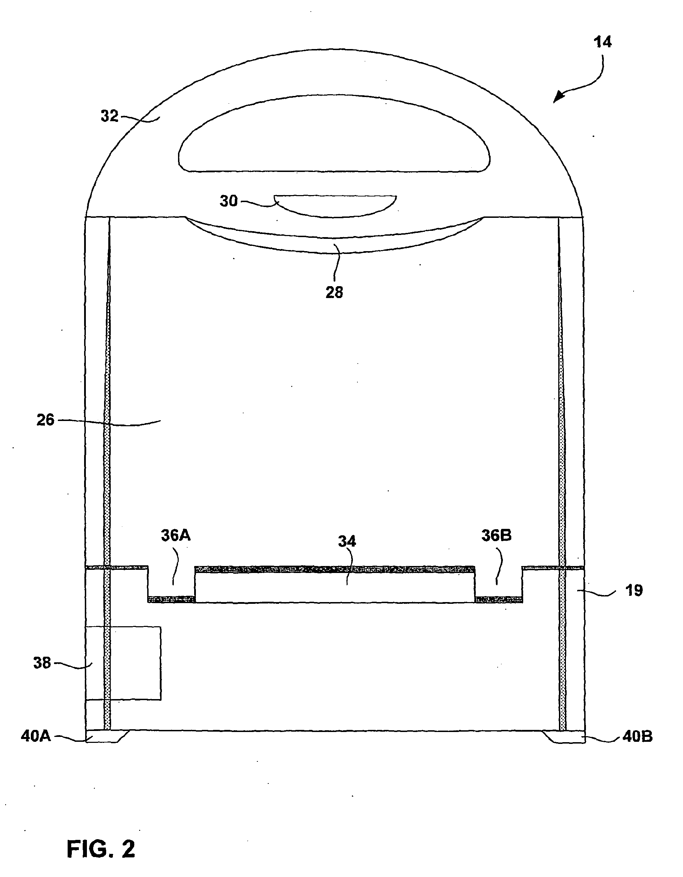

FIG. 2 is a top view of an example generator system in the screen closed configuration. The screen housing 26 is folded down in the closed position. Attached to screen housing 26 are hinges 36A and 36B and light bar 28. Pivot 34 is attached to hinges 36A and 36B to the main housing of PTD 14. A spring steel member of hinges 36A and 36B is employed to provide a moment arm about the axis formed by pivot 34 and the hinges. The moment arm provides a pop-up of screen housing 26 and allows the screen housing to remain in place when open. Button 30 releases screen housing 26 and resides at the bottom of handle 32. Also visible in this top view is the pump bay door 38 and bases 40A and 40B at the rear of PTD 14. When screen housing 26 is closed, PTD 14 is able to be moved while protecting all internal components. PTD 14 includes device housing 19 which encloses the components of the PTD.

All housing materials used in PTD 14 may be a sturdy and light material capable of providing structural support and component protection. In a preferred embodiment, the housing may be constructed of a metal such as, for example a magnesium or an aluminum alloy, but other materials may be used. These materials may include, but not be limited to, polymers such as polyurethane, or a woven polymer fabric such as those available under the trade designation Kevlar from E.I. du Pont de Nemours, Wilmington, DE. Screen housing 26 may be constructed of at least one of a magnesium alloy, an aluminum alloy, polycarbonate, polypropylene, polyurethane, polyethylene, and polystyrene.

In this configuration, screen housing 26 is resting flat against the main housing of PTD 14 and latched so that it cannot be opened. The screen is on the inside of the screen housing 26 in this illustration. Once the user pushes button 30, screen housing 26 pops up to enable the user to lift screen housing 26 and rotate it up to expose the screen. Screen housing 26 rotates along a longitudinal axis created by the interaction of hinges 36A and 36B with pivot 34. Hinges 36A and 36B may include bushings on the outer interface with the main housing to seal the main housing from any liquid ingress at the hinges. Pivot 34 may include a mechanism which creates a small moment arm on screen housing 26 when the screen housing is latched closed. When button 30 is pressed, the torque is released to move the screen housing away from the main housing of PTD 14. Pivot 34 may also include a mechanism for providing resistance against screen housing movement, once opened. This resistance may cause a user to push against screen housing 26 to create a moment arm that forces the screen housing into position. Resistance in pivot 34 also allows the screen to be placed at any angle with respect to the main housing of PTD 14.

In some embodiments, screen housing 26 may open completely after pressing button 30 by the use of a spring system or an electrical stepper motor. This lifting mechanism may also utilize a small hydraulic lift to provide enough torque to raise the screen housing. In some cases, movement may be smoothed with the use of a dampening device. A damping device may aid in a gradual start and stop to screen movement.

A three-sided light bar 28, e.g. a visual operation indicator, is located at the top of screen housing 26. While the example of FIG. 2 depicts the light bar as a concaved curved shape, the light bar may be presented in a variety of shapes. These various shapes may include a sphere, cube, rectangular cube, trapezoid, or any other polygon or rounded three dimensional shapes. This light bar may present the user with information regarding the operation of PTD 14. Due to the three-sided nature of the light bar, the user may view the light bar from a variety of locations around PTD 14

Handle 32 is positioned at the front of PTD 14 and is part of the main housing. The handle is rounded with a large hole to allow a hand of any size to carry PTD 14. Some locations on handle 32 may include ergonomic coverings to increase friction between a hand and handle 32. These coverings may also be soft to provide a comfortable interface when the user is carrying PTD 14. In some embodiments, handle 32 may be rectangular instead of curved as shown in the example of FIG. 2. In some cases, a strap or harness may be attached to handle 32 for easier carrying. This strap may be positioned over a shoulder of the user to remove a portion of the PTD load from the hand that is attached to handle 30.

At the rear of PTD 14, pump bay door 38 allows access to a replaceable fluid pump. The pump bay door 38 may be flush with the external housing and attached by a hinge along the top edge closest to the middle of PTD 14. The door may rotate up along the hinge axis to expose the pump. When closed, the door may stay closed due to friction or be secured by a mechanical latch. Alternatively, the hinge may provide resistance to pump bay door 38 opening. In some embodiments, pump bay door 38 may open along a different axis or slide back within the main housing to expose the fluid pump. Under the pump bay door 38, the pump bay may include a lip along all bay edges to keep fluid from entering the pump bay during an accidental spill on PTD 14.

Bases 40A and 40B are located at the back end of PTD 14. These may allow the device to stand on end when not in use. Bases 40A and 40B may be made out of a durable material, such as hard or soft rubber or polyurethane plastic. The material of bases 40A and 40B may measure between a 35 and 55 on a durometer. The material may absorb any impact from collisions or falls. In other embodiments, the bases may be shaped differently or connected to provide one large footing.

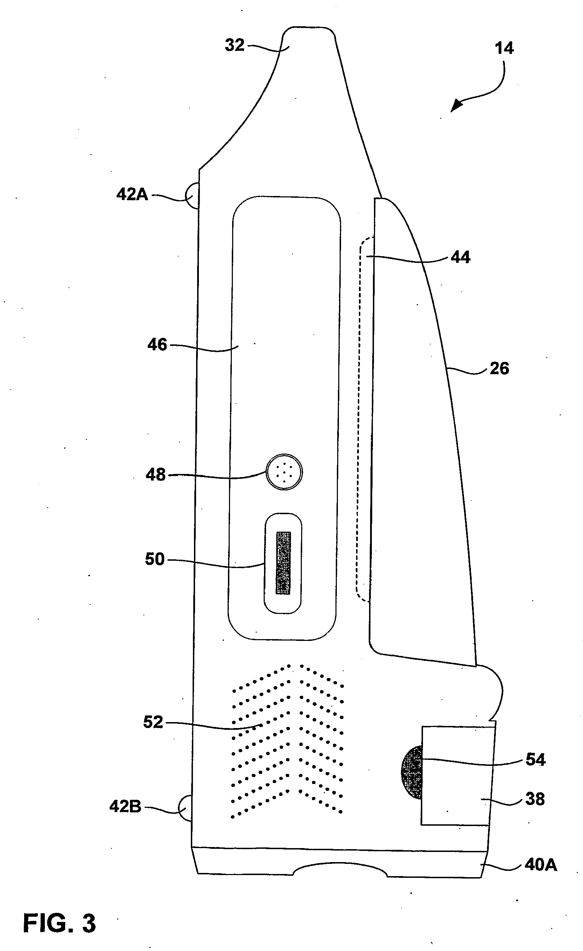

FIG. 3 is a side view of an example generator system in the screen closed configuration. The side view illustrated in FIG 3. shows screen housing 26 closed against the main housing, with recess 44 lying underneath the screen housing. Handle 32 is located at the front of PTD 14, while pads 42A and 42B are located at the bottom corners of the main housing. Inset into the main housing is connector board 46, which contains accessory port 48 and accessory port 50. Below pump bay door 38 is indent 54. Ventilation holes 52 are located along the side at the rear of PTD 14, and base 40A is attached to the rear of PTD 14.

In this example, recess 44 is only accessible when screen housing 26 is open. When the screen housing is lifted up, recess 44 may be used to hold device manuals, procedural notes, or any items that may be useful to the user. In some embodiments, recess 44 may include a clip or clips that hold a manual in position. These clips may hold down a portion of the manual or slide through the spiral binding of the manual. The clips may be able to be removed in order to read the manual closer or exchange the manual with an updated version. In another embodiment, recess 44 may include a self-adhesive label highlighting the connections necessary to operate PTD 14. This may be referred to as a quick start guide to enable the user to correctly attach the necessary components to PTD 14. Recess 44 may be one large rectangular area in the main housing, or it may be sectioned off to contain specific tools or items. In some embodiments, recess 44 may not be included in the construction of PTD 14.

Along the side is the external portion of the connector board 46. Connector board 46 is connected to the connector board port located within PTD 14. Board 46 may snap into place, require multiple screws to be secure, or be installed into the main housing by removing a section of the main housing. In some embodiments, connector board 46 may be constructed in different shapes or sizes. For example, the connector board may be oval or diamond shaped. In addition, multiple smaller connector boards may be utilized by PTD 14.

Connector board 46 may include accessory port 48 and accessory port 50 for connecting an ablation device to the connector board. Each accessory port may include a mechanism for securely attaching the associated ablation device. These mechanisms may include screws, latches, or a snap closure. While the illustrated connector board is configured for prostate ablation, many other connector boards may be exchanged to provide another therapy, diagnostic, or combination of the two procedures.

In this embodiment, accessory port 48 provides the connection between ablation device 20 and PTD 14 via cable 16. Accessory port 48 transfers the RF energy produced within PTD 14 to cable 16, and may receive therapy information such as tissue temperature as feedback. Connector 50 may be used to connect a return ground electrode that is attached to the lower back of the patient. In other embodiments, connector board 46 may include more or less accessory ports, and the accessory ports may be of any size and shape. For example, a video device for monitoring the therapy may be connected to the connector board.

In this illustration, some of the components for generating RF energy, generating the user interface, and providing power to PTD 14 may be located in the rear of the housing. For this reason, ventilation holes 52 may be included in the housing to allow heat from within the housing to escape. In some embodiments, the holes may form a different pattern and they may be of different shapes and sizes. Additionally, an exhaust fan may be placed by the holes on the inside of the housing. It should be noted that ventilation holes may be included on all or any sides of PTD 14. In particular, holes may be provides on the bottom, each side, and the rear of PTD 14. These holes may enable a steady flow of air to remove heat generated by the electrical components within PTD 14.

Indent 54 may be located just below pump bay door 38 to allow a user to open the door. The indent may allow a user to fit a finger underneath the door and pop it open. The indent 54 may instead be located at a different site along door 38. In some embodiments, indent 54 may be a button that includes a mechanism for opening the door. Alternatively, an electrical latch may be opened by using the touch screen in the screen housing when the device is operational.

The bottom of PTD 14 includes four pads 42 (42A and 42B are shown) at the four corners to support the device weight while protecting the components within PTD 14 and the surface which the device is resting on. The pads may be positioned at the four corners of PTD 14 to provide stability, and they may be spherical in shape in this embodiment. Additionally, the spherical pads may include a plurality of evenly spaced smaller spheres near the contact point of the pad to increase contact surface area. Pads 42 may be constructed of a soft or hard rubber or other durable material similar to bases 40A and 40B. Pads 42 may be compliant and such that the pads prevent PTD 14 from slipping or sliding on a level or non-level surface in which the PTD has been placed. In addition, pads 42 may not stick to the surface they contact. Pads 42 may be attached to PTD 14 by an adhesive, screw, or other fixation device.

The rear of PTD 14 is not shown, but it may contain a variety of features. An exhaust fan may be mounted within the main housing of PTD 14 to expel heat from the device though ventilation holes. A power connection may also be available for connecting the power supply to a common AC 115 Volt electrical outlet via a grounded electrical cable. Connected to the power supply may be a main power switch which is used to turn the system on and off.

Additionally, a ground terminal may be provided to electrically ground the entire system. This redundancy may be provided as a backup to the safety system provided herein. There may also be an accessory port for a floor pedal. Some users may prefer a foot operated pedal to start and stop therapy instead of, or in addition to, the controls on the hand held ablation device. A second USB port may also be provided on the back of PTD 14. In some embodiments, a network cable connection may be provided for further communications with a network or the internet.

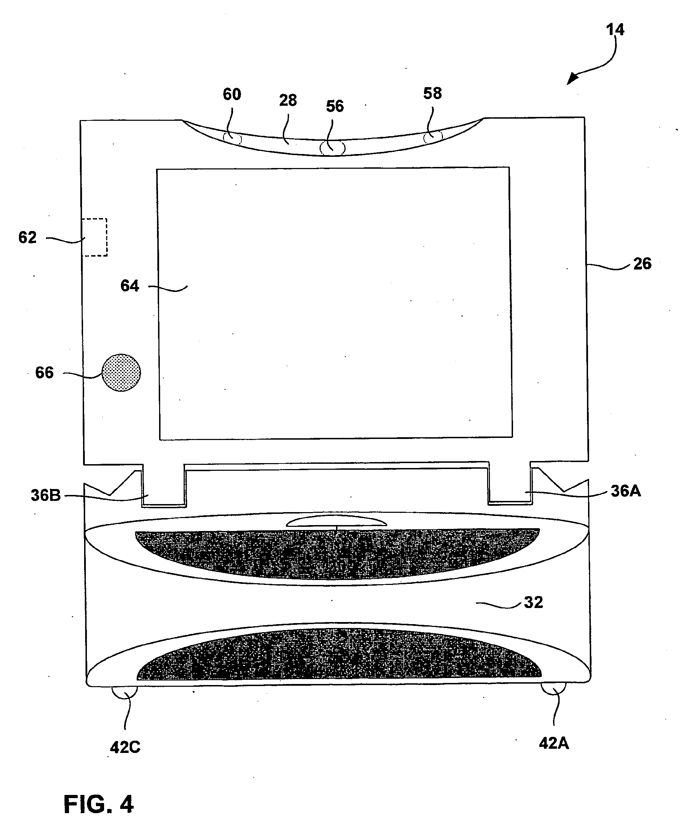

FIG. 4 is a front view of an example generator system in the screen open configuration. Open screen housing 26 includes touch screen 64, universal serial bus (USB) port, and audio speaker 66. Light bar 28 is attached to the top of screen housing 26 and includes lights 56, 58, and 60. Screen housing is attached to PTD 14 by hinges 36A and 36B, and is opened by button 30. Handle 32 allows a user to carry the device, and pads 42 (42A and 42C are shown) provide secure and stable resting points for the PTD.

Screen housing 26 may be opened to allow the physician to view touch screen 64 by pressing button 30. Button 30 is attached to a rolling latch mechanism that the downward movement of the button into lateral movement to retract the latch from the screen housing. Once this occurs, the screen may pop up a short distance to allow the user to open the screen with one hand. The screen may be left at any opening angle with respect to the closed position, and a screen housing stop may limit the opening angle. In some cases, this angle may be approximately 100 degrees from the resting position. In some embodiments, the screen may automatically open completely once button 30 is pressed. This opening may be enabled through a spring hinge or electronic motor.

Some embodiments of the screen housing 26 may include greater flexibility in screen positioning. Screen housing 26 may be mounted on a rotating hinge in which, once opened, the screen may be rotated 180 degrees in either direction. This screen rotation may allow the physician to view the screen from any location around the PTD. Other embodiments may allow further flexibility, such as a detachable screen or a wireless handheld viewing device.

Screen housing 26 may include a variety of features. Screen 64 may be a liquid crystal display (LCD) touch screen. The physician may interact with screen 64 by using a finger or stylus to touch the screen where certain icons appear. In this manner, the physician may control the therapy and PTD operation without the use of additional keyboards or pointer devices. Screen 64 may utilize any type of touch screen technology that allows the physician to select icons or graphics on the screen with a finger, stylus, or latex gloved finger.

Screen 64 may utilize a resistive system to detect the location of a touch on the screen. The resistive system consists of a normal glass panel that is covered with a conductive and a resistive metallic layer. The conductive and resistive layers are separated by spacers with a scratch-resistant layer disposed on the surface of screen 64. An electrical current flows through the conductive and resistive layers when screen 64 is operational. When the physician touches the screen, the conductive layer contacts the resistive layer on the location of the touch. The change in the electrical field is detected by screen 64 and the coordinates of the location is calculated by a processor. Once the coordinates are calculated, a driver translates the location into data that the operating system uses to control system 14.

In some embodiments of screen 64, screen 64 may utilize a capacitive system. The capacitive system includes a capacitive layer that stores electrical charge that is placed on a glass panel of screen 64. When the physician touches the monitor with a finger, a portion of the electrical charge is transferred to the physician. This transfer of electrical charge reduces the charge in the capacitive layer. A plurality of circuits located at each corner of screen 64 measures the decrease in charge, and a processor calculates the location of the touch from the relative differences in electrical charge at each corner of the screen. Screen 64 may be brighter when using the capacitive system as compared to the resistive system, but insulating objects may not be detected by the screen.

In alternative embodiments, screen 64 utilizes a surface acoustic wave system to detect touch on the screen. Two transducers, one receiving transducer and one sending transducer, are placed along an x axis and a y axis of the glass plate of screen 64. A plurality of reflectors are also placed on the glass plate to reflect an electrical signal sent from one transducer to the other transducer. The receiving transducer detects any disturbance in the sending wave from a touch to screen 64 and determines the location of the disturbance. The surface acoustic wave system contains no metallic layers, which allows almost all light to be delivered from screen 64 to the physician.

Adjacent to screen 64 is speaker 66. Speaker 66 may deliver audible tones or voice cues related to PTD operation or therapy progress. The volume of speaker 66 may be adjusted by touch screen 64 or a small dial on the side of screen housing 26. On one side of screen housing 26, a USB port 62 may be included for the transfer of data between PTD 14 and another computing device. In the preferred embodiment, USB port 62 may be located on the side of PTD 14 opposite to connector board 46 to keep USB port 62 separate from therapy connections. In some embodiments, a video camera may be located within screen housing 26.

In other embodiments, screen housing 26 may include other communication devices different than a USB port 62. For example, screen housing 26 may include an IEEE 1394 port, a serial port, a video output, a video input, a microphone, or an audio output. Alternatively, screen housing may contain a wireless communication antenna. The antenna may be completely inside screen housing 26 or protruding outside of the screen housing. The wireless communication antenna may provide communication via protocols such as 802.11 a, 802.11 b, 802.11 g, or Bluetooth. Other protocols may include the medical implant communication system (MICS) or the medical implant telemetry system (MITS) that operate at a frequency between 402 and 405 megahertz.

Light bar 28 is located at the top of screen housing 26. The light source within light bar 28 may be one or more colored lights. These lights may include electric light bulbs, light emitting diodes (LEDs), light pipes, or any other device that emits visible light. Any number of light sources may be used, and they may each emit one or more wavelength of light, or color. In one embodiment, three LEDs may be used beneath the translucent light bar covering. Power light 56 may be green in color and illuminate when PTD 14 power is on. Therapy lights 58 and 60 may be blue in color and illuminate when therapy is being delivered. These light sources cause light bar 28 to glow when they are illuminated. Lights may continue to illuminate when screen housing 26 is closed.

In some embodiments, the light sources may blink at certain times. For example, therapy lights 58 and 60 may begin to blink when therapy is ready to be delivered. In some cases, the lights may be able to change color to indicate therapy progress or warn the physician of a problem. For example, lights 58 and 60 may begin to flash red in color if a device becomes disconnected or the therapy reaches unsafe levels for the patient.

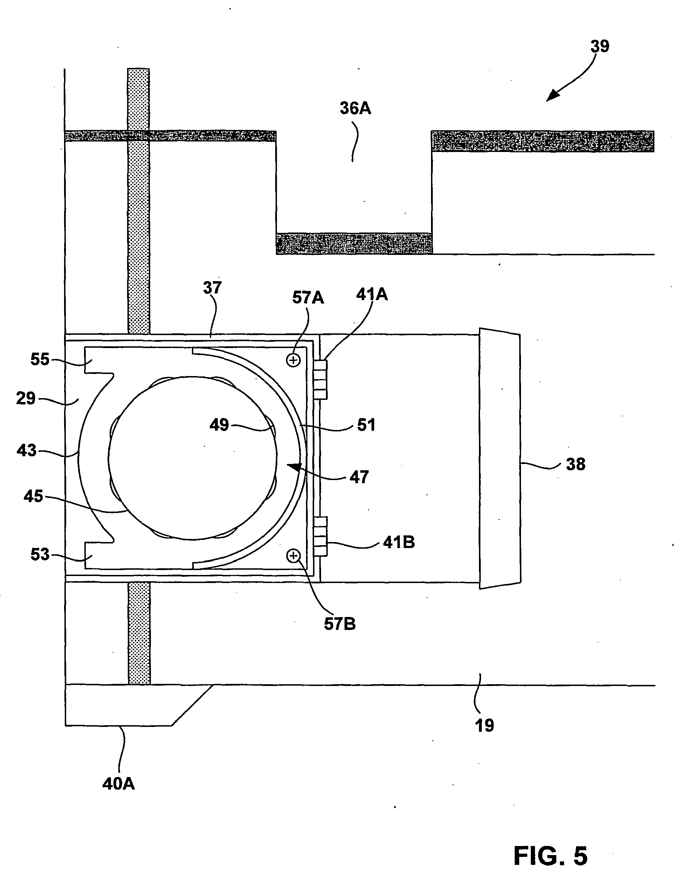

FIG. 5 is a top view of a peristaltic fluid pump in an open pump bay of an example generator system. As shown in FIG. 5, PTD 39 is an alternative embodiment of PTD 14. The partial view of PTD 39 includes opened pump bay door 38 attached to device housing 19 via hinges 41A and 41B. Pump bay 29 also includes channel 37 around the upper edge of the pump bay. Fluid pump 43 is disposed within pump bay 29 and attached to the pump bay via securing mechanisms 57A and 57B. Fluid pump 43 includes rotor 45, tube channel 47, bearings 49, tube cover 51, input 53 and output 55. Fluid pump 43 also includes a cable to electrically couple the fluid pump to control circuitry of PTD 39.

Pump bay 29 is an opening within device housing 19 large enough to accept fluid pump 43 and allow pump bay door 38 to lie flush with the device housing when the pump bay door is in the closed configuration. Channel 37 is disposed just inside of the perimeter of pump bay 29. Channel 37 directs, or channels, uncontained fluid on device housing 19 that flows toward pump bay 29 away from entering the interior of the pump bay. An uncontained fluid may be water, saline, alcohol, blood, or any other fluid that may come into contact with PTD 39.

Pump bay door 38 rotates about a longitudinal axis of hinges 41A and 41B when the physician or other user lifts the pump bay door from the closed configuration into the open configuration. As shown in FIG. 5, Pump bay door 38 may lock closed with a latch, snap fit, or other locking mechanism. In some embodiments, pump bay door 38 may mate with a rubber seal around the perimeter of pump bay 29 such that fluid pump 43 is protected from any uncontained fluid that comes into contact with device housing 19. In other embodiments, hinges 41A and 41 B may include springs that provide a moment arm bias to keep pump bay door 38 open when the door is not locked in the closed configuration.

Fluid pump 43 is a peristaltic pump that does not come into contact with the fluid being pumped. A flexible tube (such as tube 18 from FIG. 1) includes an inflow opening placed within a first container and an outflow opening opposite the inflow opening. The outflow opening is attached to a therapy device that delivers the fluid. A middle section of flexible tube is placed within tube channel 47, between rotor 45 and tube cover 51. In this embodiment, the inflow opening end of the flexible tube is located in the direction of input 53 and the outflow opening is located in the direction of output 55.

PTD 39 operates pump 43 by rotating rotor 45 in a counter-clockwise direction to move fluid forward within the flexible tube from input 53 to output 55. As rotor 45 rotates, bearings 49 roll over the flexible tube and displace fluid within the flexible tube in the direction of the rotor. Rotor 45 may also rotate in the clockwise direction to move fluid in the reverse direction. The fluid delivery rate produced by fluid pump 43 is a function of the inner diameter of the flexible tube and the rotational speed of rotor 45. In some embodiments, the flexible tube may include more than one tube sections. For example, the flexible tube within tube channel 47 may connect to a separate inflow tube and an outflow tube.

Securing mechanisms 57A and 57B secure fluid pump 43 within pump bay 29. In the embodiment of FIG 5, mechanisms 57A and 57B are Phillips head screws that are inserted through a base of fluid pump 43 to the interior of pump bay 29. Pump bay 29 may include threaded holes within a bracket or mounting piece that accept securing mechanisms 57A and 57B. In alternative embodiments, securing mechanisms other than screws may be used. For example, pins, latches, sliding guides, or another type of mechanism may secure fluid pump 43 within pump bay 29.

A user, such as a field technician or the physician, may remove fluid pump 43 in the event that the fluid pump fails or a different fluid pump is needed within PTD 39. Being able to remove and replace fluid pump 43 allows PTD 39 to be used with a variety of therapies or to be upgradeable as system components improve to better treat patient 12.

Fluid pump 43 may pump fluid in a different manner than described with respect to the peristaltic pump. Possible types of other positive displacement pumps may include an internal gear pump, and external gear pump, a vane pump, a flexible member pump, a lobe pump, a circumferential piston pump, or a screw pump. While these pumps are rotary pumps, reciprocating pumps may be used in some embodiments. In alternative embodiments, dynamic or centrifugal pumps may also be used as fluid pump 43.

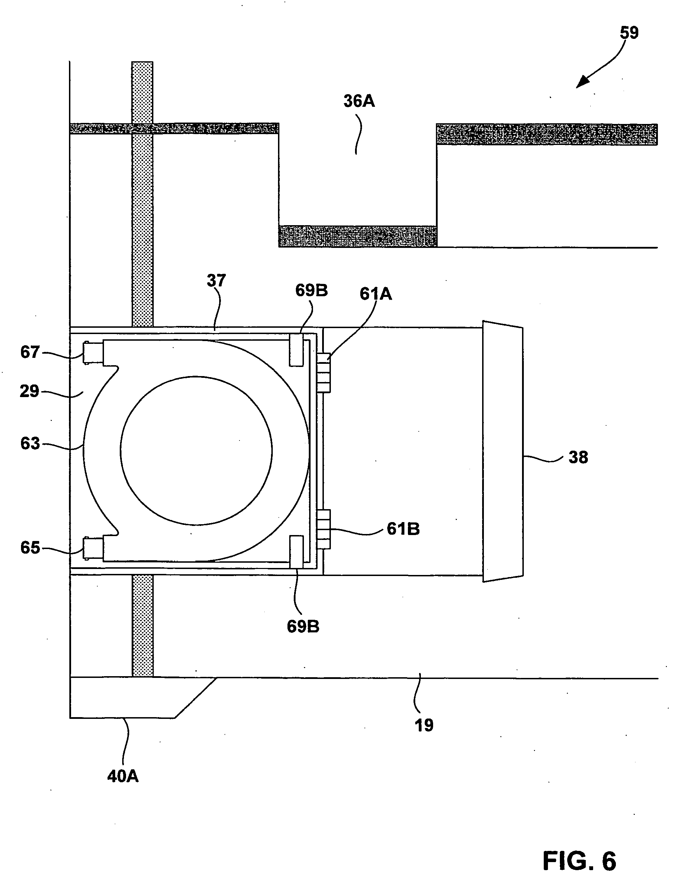

FIG. 6 is a top view of a internal gear fluid pump in an open pump bay of an example generator system. As shown in FIG. 6, PTD 59 is an alternative embodiment of PTD 14 and 39. The partial view of PTD 59 includes opened pump bay door 38 attached to device housing 19 via hinges 61A and 61B. Pump bay 29 also includes channel 37 around the upper edge of the pump bay, similar to PTD 39. Fluid pump 63 is disposed within pump bay 29 and attached to the pump bay via securing mechanisms 69A and 69B. Fluid pump 63 includes input 65 and output 67. Fluid pump 63 also includes a cable to electrically couple the fluid pump to control circuitry of PTD 59.

Pump bay 29 is an opening within device housing 19 large enough to accept fluid pump 63 and allow pump bay door 38 to lie flush with the device housing when the pump bay door is in the closed configuration. Channel 37 is disposed just inside of the perimeter of pump bay 29. Pump bay door 38 rotates about a longitudinal axis of hinges 61A and 61B when the physician or other user lifts the pump bay door from the closed configuration into the open configuration. Pump bay door 38 may lock closed with a latch, snap fit, or other locking mechanism. In some embodiments, pump bay door 38 may mate with a rubber seal around the perimeter of pump bay 29 such that fluid pump 63 is protected from any uncontained fluid that comes into contact with device housing 19. In other embodiments, hinges 61A and 61B may include springs that provide a moment arm bias to keep pump bay door 38 open when the door is not locked in the closed configuration.

Fluid pump 63 is an internal gear pump that fully encloses the fluid being pumped into and out of the fluid pump. Fluid pump 63 includes gear teeth that carry fluid from input 65 to output 67. An input tube (not shown) connects a fluid container to input 65, and an output tube (such as tube 18 from FIG. 1) connects output 67 to a therapy device. An outer gear of fluid pump 63 drives an inner gear on a stationary pin. The outer and inner gears create voids as they move out of mesh, or when teeth are interlocked, and the fluid flows into the cavities between the gear teeth. As the outer and inner gears come back into mesh, the volume of the cavities is reduced and the fluid is forced out of, or discharged from, output 67. In addition, a crescent shaped barrier between the outer and inner gears prevents the fluid from flowing backwards against the direction of the gears. The fluid delivery rate from output 67 is determined by the size are rotational speed of the inner and outer gears. In some embodiments, the internal gear setup may vary from fluid pump 63 described herein.

The input tube and output tube may attach to their respective input 65 and output 67 via a lure-lock connector. A female lure-lock connector is attached to the ends of each input tube and output tube. Each female lure-lock connector may then attach to the male lure-lock connector located at the end of each input 65 and output 67. Each female lure-lock connector may be rotated to screw onto each male lure-lock connector and securely fasten each tube to fluid pump 63. In some cases, a locking mechanism may not be necessary for a connection, but the locking connection may prevent tube disconnection when fluid pump 63 is used to produce high pressures.

Securing mechanisms 69A and 69B secure fluid pump 63 within pump bay 29. In the embodiment of FIG 6, mechanisms 69A and 69B are latches that snap over a base of fluid pump 63 to hold the pump in place. Mechanisms 69A and 69B may include springs that provide bias against the base. In alternative embodiments, securing mechanisms other than latches may be used. For example, pins, screws, sliding guides, or another type of mechanism may secure fluid pump 63 within pump bay 29.

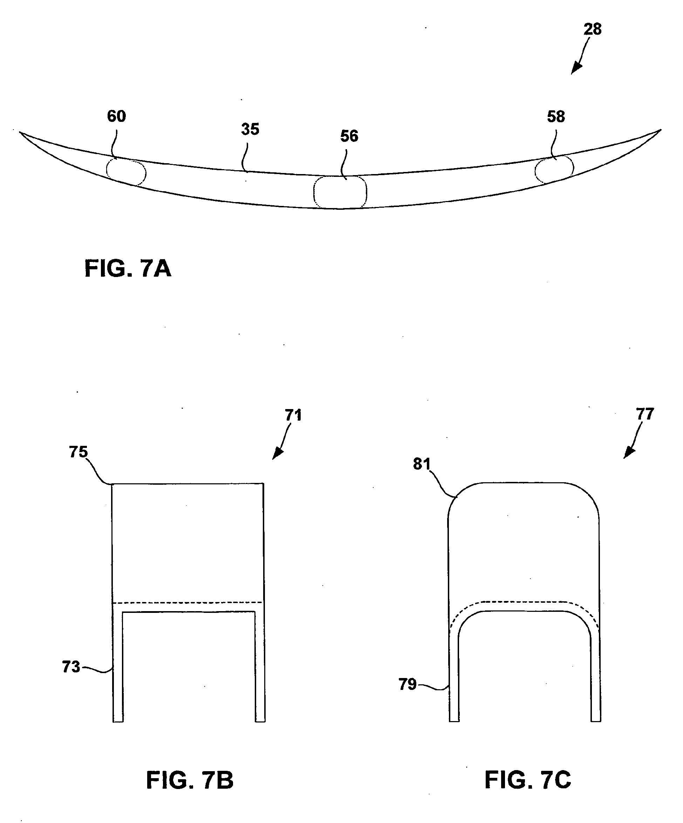

FIG. 7A is an enlarged side view of an example light bar of an example generator system. As shown in FIG. 7A, light bar 28 is a visual operation indicator that includes cover 35, power light 56, and therapy lights 58 and 60. As mentioned previously, power light 56 visually indicates a system power status and therapy lights 58 and 60 visually indicate a therapy delivery status to the physician. In some embodiments, light bar 28 may secure two pieces, or more than two pieces, of screen housing 26.

Lights 56, 58 and 60 may include any of electric light bulbs, light emitting diodes (LEDs), light pipes, or any other device that emits visible light. In the embodiment of FIG. 7A, power light 56 is green in color and illuminates when PTD 14 is operational, e.g. the power is on. Power light 56 emits light at a wavelength between 491 nanometers (nm) and 575 nm. Therapy lights 58 and 60 each produce a blue light when ablation device 20 transmits RF energy to patient 12. Each of therapy lights 58 and 60 emit blue light of a wavelength between 424 nm and 491 nm. In some embodiments, lights 56, 58 and 60 may emit light of different wavelengths, or colors. In other embodiments, lights 56, 58 or 60 may blink on and off at a certain rate to indicate a malfunction or other need that the physician needs to address. Alternatively, light bar 28 may include more or less lights to visually indicate power status or delivery status to the physician.

Cover 35 encloses lights 56, 58 and 60 against screen housing 26. Cover 35 is constructed out of a translucent material, or a material that allows at least a portion of the light from lights 56, 58 and 60 to pass through the cover. In a preferred embodiment, the translucent material of cover 35 disperses the emitted light to simulate a glow. This softer light may be easier for the physician to look at than direct light through a clear cover 35. However, cover 35 may be completely clear and transmit 100 percent of the emitted light in some embodiments The material of 35 may include polycarbonate, polypropylene, polyurethane, polytetrafluoroethylene, polyacetylene, polyethylene, polystyrene, or some combination of these materials. Other light transmitting materials may also be used in cover 35.

Cover 35 also includes structure that allows the cover to manipulate a diffusion pattern of the emitted light. Cover 35 may include ribbing or other structures inside of the cover that separate the emitted light from lights 56, 58 and 60. Cover 35 may also include a formation or cutout that allows a message to glow when light is being emitted. For example, cover 35 may include windows, icons, images, pictures, text, or other shapes extruded or printed onto the cover. In an example embodiment, light 56 may produce a glowing word "ON" when the system has power. Cover 35 may produce other lighting effects through the use of mirrors, prisms, and other light absorbing or light reflecting materials.

Cover 35 also includes a curved top surface that is higher at each side than in the middle. The curve of the curved top has a radius generally between 15 cm and 102 cm (6 inches and 40 inches). Specifically, the curve has a radius between 35-60 cm (14 inches and 24 inches). Each side of cover 35 is perpendicular to the curved top. The sides are also curved in the same direction as the curved top, but the curve of each side has a slightly smaller radius than the radius of the curved top. In some embodiments, the curved top of cover 35 may curve in the opposite direction, and each side would also curve in the opposite direction shown in FIG. 7A.

FIGS. 7B and 7C are enlarged end views of two example light bars with slightly different shapes. FIG. 7B shows an example cover 71, which is an embodiment of cover 35. Cover 71 includes sides 73 that are normal to the top of the cover. Corners 75 are at a right angle and form an edge where sides 73 meet the top of cover 71. The dotted line indicates the top of cover 71 at the middle length of the cover.

FIG. 7C shows an example cover 77, which is an embodiment of cover 35. Cover 77 includes sides 79 that are normal to the top surface of the cover. Corners 81 are curved to connect sides 79 with the op of cover 77. Curved corners 81 may provide a rounded edge that allows a more continuous emission of light from cover 77 when compared to corners 75 of cover 71. In some embodiments, sides 73 or 79 may not be perpendicular to the top of covers 71 or 77, respectively. For example, sides 73 may be at an angle greater than 90 degrees with respect to the top of cover 71, such that the distance between the sides is greater at the bottom of cover 71 than the distance between the sides at the top of cover 71.

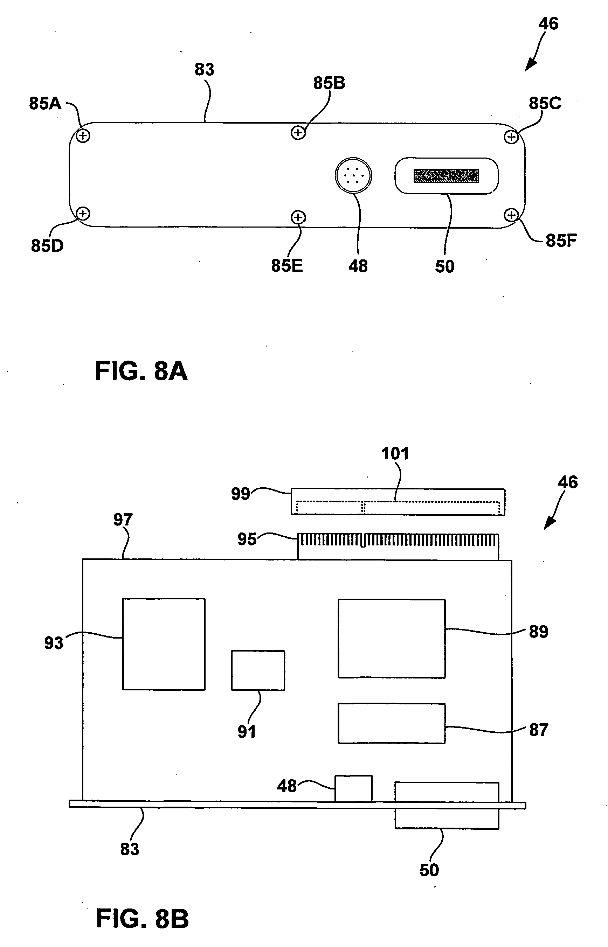

FIG. 8A is an enlarged side view of an example removable connector board. As shown in FIG. 8A, connector board 46 includes face plate 83, securing mechanisms 85A-85F (collectively securing mechanisms 85), accessory port 48 and accessory port 50. Face plate 83 mates against device housing 19 to prevent PTD 14 internal circuits and mechanisms from being damaged during use.

In the example of FIG. 8A, securing mechanisms 85 are screws that lock into helical tapped holes of device housing 19. More or less securing mechanisms may be used in connector board 46. Securing mechanisms 85 also coupled secures connector board 46 into the connector board port 99 (shown in FIG. 8B) that is electrically coupled to a motherboard of PTD 14 to enable the PTD operation. In this manner, connector board 46 is removably coupled to connector board port 99. In some embodiments, securing mechanisms other than screws may be used to secure connector board 46. For example, one or more latches or clips may be used instead of screws. Connector board 46 may also slide into tracks within device housing 19 and snap into place.

As described above, accessory port 48 transfers RF energy generated by a signal generator within PTD 14. The signal generator is a specific type of energy source that may be within PTD 14. Accessory port 50 provides an attachment for a ground electrode. Another accessory port may be provided to attach a video monitoring device. In other embodiments, connector board 46 may include more or less accessory ports than accessory ports 48 and 50. For example, connector board 46 may only have an antenna if the connector board is designed to communicate with other devices. Other examples include connector board 46 including a plurality of accessory ports to support a 12-lead electrocardiogram (ECG) when the connector board is designed to diagnose cardiac dysfunction. PTD 14 may perform the function of delivering a therapy, presenting therapy data to a user or another device, or communicate directly with an external or implanted device. Some alternative peripheral accessories to ablation device 20 may include an ultrasound paddle, a communication antenna, or a battery recharging device. In other embodiments, connector board may support portable media slots; e.g. compact disks (CD) or digital versatile disk (DVD), a universal serial bus (USB) port, or any other port that allows PTD 14 to communicate with another media or device.

FIG. 8B is a top view of an example removable connector board. As shown in FIG. 8B, connector board 46 includes face plate 83, accessory ports 48 and 50, circuit board 97, multiplexer 87, processor 89, memory controller 91 and memory 93. Connector board 46 also includes multi-pin connector 95. Connector board port 99 includes slot 101 that accepts multi-pin connector 95, wherein connector board port is coupled to a motherboard or processor of PTD 14. Connector board 46 does not include an enclosure that surrounds the connector board, but other connector boards may include an enclosure that would create a sealed module that is removable from connector board port 99 and PTD 14.

Circuit board 97 electrically couples the components of connector board 46. Circuit board 97 is a printed circuit that may also provide structural rigidity to hold each component. Multiplexer 87 controls the electrical signals from processor 89 to accessory ports 48 and 50. Processor 89 processes information from a motherboard of PTD 14 and uses instructions stored in memory 93 to deliver the appropriate electrical signals to ablation device 20. Memory 93 may also store data related to the operation of ablation device 20 and data related to identifying the type or identity of the ablation device connected to connector board 46.

In some embodiments, connector board 46 may also include a device identity sensor that recognizes ablation device 20. Processor 89 may then perform some function based upon the recognized device identity sensor. Processor 89 may enable a therapy, enable a test program that diagnoses PTD 14, or allow patient 12 data to be transferred to another device. Processor 89 may also then load software associated to the recognized ablation device 20. In other embodiments, memory 93 may recognize that the particular ablation device 20 has been used previously and prevent the physician from using the ablation device because a new ablation device should be used for patient 12. The device identity sensor provides a mechanism, similar to a key, that enables a user to perform certain functions with PTD 14.

In alternative embodiments, connector board 46 may not include a separate processor to control the operation of the connector board. In these embodiments, connector board 46 may not include any processing circuitry, as the connector board may only transfer electrically signals by a motherboard or other circuitry within PTD 14. In addition, the motherboard may detect which type of connector board is electrically coupled to connector port 99.

Multi-pin connector 95 may be constructed in a different configuration to connect PTD 14 and connector board 46. For example, multi-pin connector 95 may include a four-pin snap connector. Any connector may be used based upon whether connector board 46 utilizes digital or analogue signals, or both.

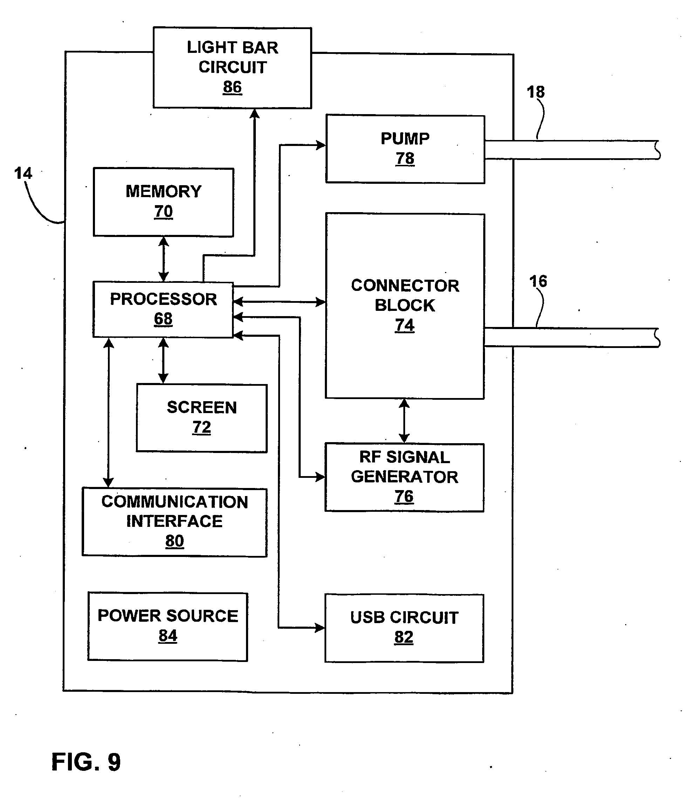

FIG. 9 is functional block diagram illustrating components of an exemplary generator system. In the example of FIG. 9, PTD 14 includes a processor 68, memory 70, screen 72, connector block 74, RF signal generator 76, pump 78, telemetry interface 80, USB circuit 82, power source 84, and light bar circuit 86. As shown in FIG. 9, connector block 74 is coupled to cable 16 for delivering RF energy produced by RF signal generator 76. Pump 78 produces pressure to deliver fluid through tube 18.

Processor 68 controls RF signal generator 76 to deliver RF energy therapy through connector block 74 according to therapy parameter values stored in memory 70. Processor 68 may receive such parameter values from screen 72 or telemetry interface 80 or USB circuit 82. When signaled by the physician, which may be a signal from the ablation device 20 conveyed through connector block 74, processor 68 communicates with RF signal generator 76 to produce the appropriate RF energy. As needed, pump 78 provides fluid to irrigate the ablation site or provides fluid to the electrode during wet electrode ablation.

In a preferred embodiment, the RF signal generator may have certain performance parameters. In this exemplary case, the generator may provide RF energy into two delivery channels with a maximum of 50 Watts per channel. Other embodiments may include generation in excess of 100 watts for one channel. Duty cycles of the energy may alter the total power capable of being produced. In other examples, the ramp time for a 50 Watt change in power may occur in less than 25 milliseconds, and the output power may be selected in 1 Watt steps. The maximum current to be provided to the patient may be 2 Amps, and the maximum voltage may be 180 Volts. Other embodiments of the signal generator may have different power capabilities as needed by the intended use of PTD 14.

Connector block 74, e.g. connector board 46, may contain an interface for a plurality of connections, not just the connection for cable 16. These other connections may include one for a return electrode, a second RF energy channel, or a separate temperature sensor. As mentioned previously, connector block 74 may be a variety of blocks used to diagnose or treat a variety of diseases. All connector blocks may be exchanged and connect to processor 68 for proper operation. Pump 78 may be replaceable by the physician to replace a dysfunctional pump or use another pump capable of pumping fluid at a different flow rate.

Processor 68 may also control data flow from the therapy. Data such as RF energy produced, temperature of tissue, and fluid flow may be channeled into memory 70 for analysis. Processor 68 may comprise any one or more of a microprocessor, digital signal processor (DSP), application specific integrated circuit (ASIC), field-programmable gate array (FPGA), or other digital logic circuitry. Memory 70 may include multiple memories for storing a variety of data. For example, one memory may contain therapy parameters, one may contain PTD operational files, and one may contain therapy data. Memory 70 may include any one or more of a random access memory (RAM), read-only memory (ROM), electronically-erasable programmable ROM (EEPROM), flash memory, or the like.

Processor 68 may also send data to USB circuit 82 when a USB device is present to save data from therapy. USB circuit 82 may control both USB ports in the present embodiment; however, USB circuit 82 may control any number of USB ports included in PTD 14. In some embodiments, USB circuit may be an IEEE circuit when IEEE ports are used as a means for transferring data.

The USB circuit may control a variety of external devices. In some embodiments, a keyboard or mouse may be connected via a USB port for system control. In other embodiments, a printer may be attached via a USB port to create hard copies of patient data or summarize the therapy. Other types of connectivity may be available through the USB circuit 82, such as internet access.

Communications with PTD 14 may be accomplished by radio frequency (RF) communication or local area network (LAN) with another computing device or network access point. This communication is possible through the use of communication interface 80. Communication interface 80 may be configured to conduct wireless or wired data transactions simultaneously as needed by a user, e.g., a physician or clinician. In some embodiments, communication interface 80 may be directly connected to connector block 74.

PTD 14 may communicate with a variety of device to enable appropriate operation. For example, PTD may utilize communication interface 80 to monitor inventory, order disposable parts for therapy from a vendor, and download upgraded software for a therapy. In some embodiments, the physician may communicate with a help-desk, either computer directed or human staffed, in real-time to solve operational problems quickly. These problems with PTD 14 or a connected ablation device may be diagnosed remotely and remedied via a software patch in some cases.

Screen 72 is the interface between PTD 14 and the physician. Processor 68 controls the graphics displayed on screen 72 and identifies when the physician presses on certain portions of the screen 72, which is sensitive to touch control. In this manner, screen 72 operation may be central to the operation of PTD 14 and appropriate therapy or diagnosis.

Processor 68 also determines the operation of light bar circuit 86. In the present embodiment, processor turns on a green light when PTD power is on while blue lights are illuminated when therapy is being delivered. Processor 68 may be capable of controlling any number of different lights which illuminate light bar 28.

Power source 84 delivers operating power to the components of PTD 14. Power source 84 may utilize electricity from a standard 115 Volt electrical outlet or include a battery and a power generation circuit to produce the operating power. In other embodiments, power source 84 may utilize energy from any outlet that provides between 100 and 240 Volts. In some embodiments, the battery may be rechargeable to allow extended operation. Recharging may be accomplished through the 115 Volt electrical outlet. In other embodiments, traditional batteries may be used.

In some embodiments, signal generator 76 may be a different type of energy source. For example, the energy source may convert power from power source 84 to produce steam, mechanical energy, or any other type of output that may perform work on patient 12. Other energy may be laser energy or ultrasound energy. In this manner, the energy source may produce electrical, chemical, or mechanical energy.

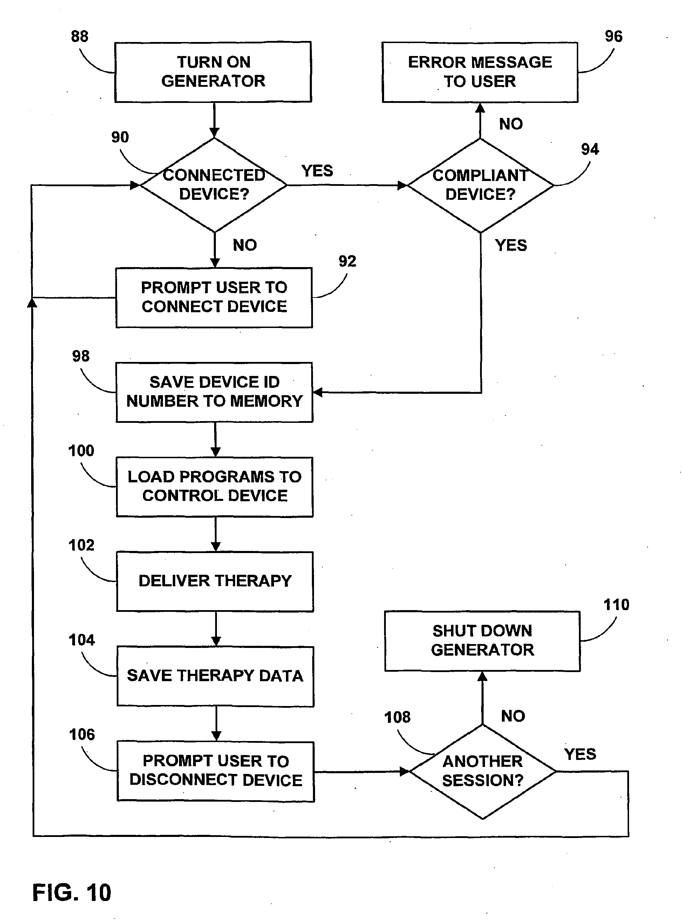

FIG. 10 is a flow diagram illustrating an example technique for operating the generator system in attaching a ablation device and providing therapy to a patient. In the example of FIG. 10, system 10 delivers therapy. In another embodiment, system 10 may diagnose a condition, or diagnose a condition and provide an associated therapy.

In this embodiment, a physician (a user) turns on the generator (88). Once the system is powered up, the system looks for a connected device (90). If a device is not connected, a prompt is given to the user to connect a device (92). Once a device is connected, the system checks the device to determine if it is compliant with the system (94). If it is not, an error message may be issued to the user indicating that the device is not compliant with the system (96).

If the device is compliant, the system records the device identification number (ID) to memory so that the system may log the use of that device (98). The PTD may then load the associated software to operate the therapy of the connected device (100). Once the software is loaded, the user may begin to deliver therapy to the patient (102). After therapy is concluded, therapy data may be saved to the memory of PTD 14 and to a smart memory chip within the connected ablation device (104). With therapy data contained within the device, the device could be examined at a later date for quality control or therapy investigation reasons.

After data has been saved, the system may prompt the user to disconnect the ablation device (106). Once disconnected, the system may wait for the user to begin another therapy session (108). If another session is desired, the system begins again with block 90. If no new session is desired, the generator may shut down (110).

This example of system operational flow is only an example, and other embodiments may be different. For example, the user may have much more flexibility in operation instead of being forced to the next step in therapy. The order of steps may also be rearranged depending on the user's preference or the therapy being delivered. The system may also enable a phantom operation mode to train new users on the system. In this case, a device may not be connected, or the connected device may be non-functional.

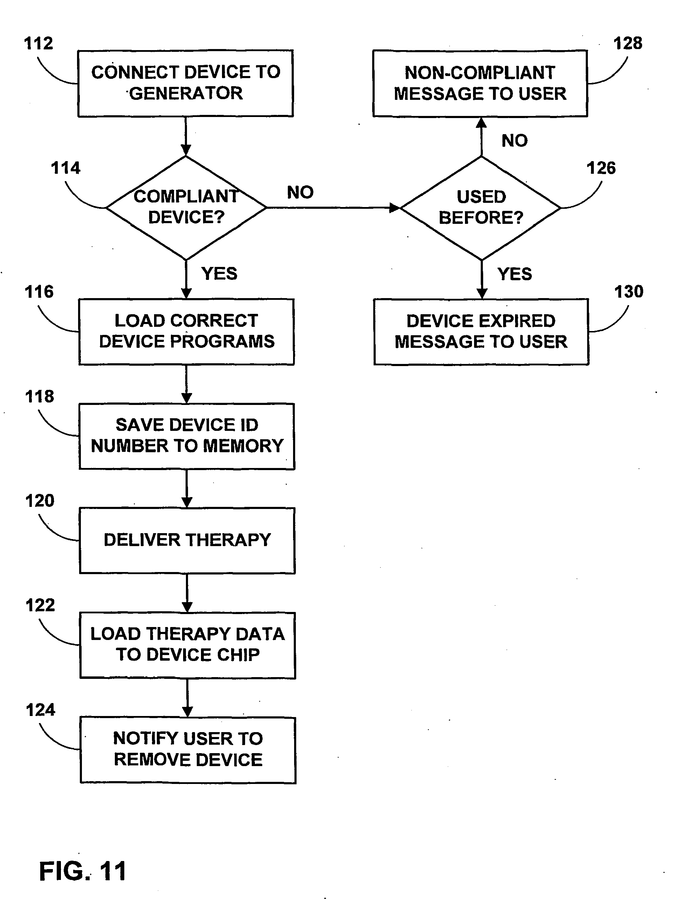

FIG. 11 is a flow diagram illustrating an example technique for identifying a connected ablation device and determining its status before providing therapy to a patient. In the example of FIG. 11, a ablation device is connected to the system in order for therapy to be delivered. The device is connected to the system, the generator in this case, by the user (112). Immediately, the system interrogates the connected ablation device to determine if it is compliant (114).

If the device is compliant, the system continues by loading the associated programs to control the connected ablation device (116). If the device does not comply with the system, the system determines if the device has been used before (126). This may be determined by either locating data within a smart memory chip of the ablation device or locating the ablation device ID within the PTD and any associated data. If the device has not been used before, but it is still not compliant with the system, a non-compliant message may be delivered to the user (128). If the device has been used before, an expiration message may be delivered to the user (130). In some cases, device may only be used once, with one patient. In other cases, a device may be used with a plurality of patients, but the operational life of the ablation device is limited to a set number of uses. For this reason, a device may be expired after a predetermined number of uses. In other embodiments, the smart memory chip may deem a device expired when it become dysfunctional due to a mechanical or electrical failure.

If an acceptable ablation device is connected, the ID number of the ablation device is saved to memory once the software is loaded (118). The user is then able to perform any appropriate therapy with the system (120). After therapy is concluded, a log of data encompassing the therapy delivered is loaded into the smart memory chip of the ablation device (122). Once this is completed, the user may be notified that the system is ready for the device to be removed (124).

In some embodiments, more involved operations may govern the use of ablation devices. For example, the system may check for older versions of ablation devices or determine the status of a device when multiple uses are acceptable. In other embodiments, a variety of error messages may be issued to the user. These error messages may even suggest possible methods to troubleshoot a malfunctioning device which should be compliant.

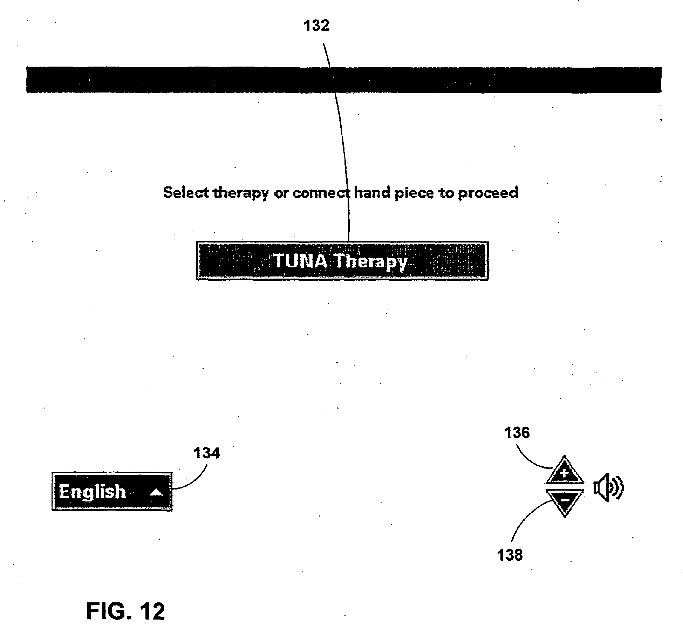

FIG. 12 is an exemplary screen shot of the main menu provided by the user interface. All boxed items in the following screens are interactive, meaning that the user may touch that portion of the screen to select that item. Although the following sample screen shots are used in this embodiment, any number of variations may be made to this graphic interface as ablation devices, diagnosis devices, or functionality are modified within the system.

In this main menu, a few options reside for the user. Therapy box 132 indicates that "TUNA Therapy," or prostate ablation, would be delivered if the user pressed box 132. In other embodiments, an plurality of therapy boxes may be present, depending on the device or devices connected to PTD 14. When the user selects one of the boxes, that program is initialized.

Language box 134 may reside at the lower left hand corner of the screen. The selected language may be indicated, as English is shown in box 134. If the user desires to change the language in the user interface, pressing the box may bring up another menu which includes other supported languages. Selecting one of those languages displayed may immediately change the language used in the interface. In some embodiments, English may always be the default language, while other embodiments may save the default language as the last selected language from box 134.

Volume may also be modified on the main menu screen. Volume up triangle 136 may increase the volume one level for each time it is selected. Alternatively, volume triangle 138 may decrease the volume one level for each time it is pressed. Upon a volume change, an audible note may be played at the newly selected volume level. In some embodiments, a numeric indicator of the volume level may be shown for a certain period of time upon a volume change. In other embodiments, the shape of triangle 136 may be a square, circle, oval, or any other shape.

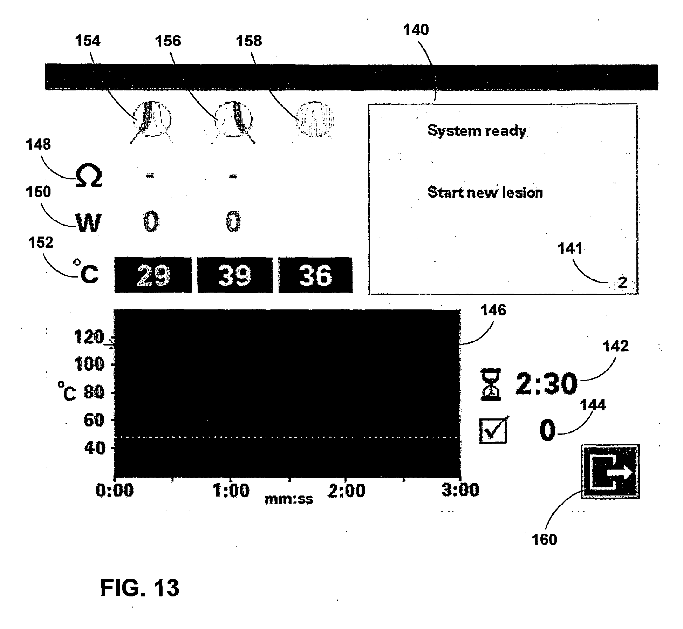

FIG. 13 is an exemplary screen shot of the delivery screen when the system becomes operational. Before the physician begins therapy, this screen displays the delivery information. Message box 140 indicates that the system is ready for the physician to begin therapy. Indicator 141 is associated with message box 140 and provides a reference to the user in case the user desires to further investigate the message or error in message box 140. In some embodiments, the manual may be printed in more languages than the user interface supports. If needed, the user may use the indicator to identify the message of message box 140 in a particular language.

Timer 142 indicates the time remaining for the therapy. Since the therapy has not begun, two minutes and thirty seconds remain for therapy. Check box 144 indicates how many lesions, or ablation areas, have been completed. Graph 146 displays the temperature of the tissue with respect to time. The dotted line may indicate the threshold safe temperature for the urethra. At approximately 115 degrees Celsius, an arrow indicates the target temperature for the tissue to be ablated.

Omega symbol 148 indicates the units of resistance, in Ohms, of the tissue between the anode and cathode for each tissue area. Letter W 150 indicates the power, in Watts, of the RF energy being delivered to each needle. Degree C 152 indicates the temperature, in degrees Celsius, of each ablation site and the urethra. In some embodiments, these indicators may be in different units as requested by the therapy or the user. As other therapies are used, other measurable may be used to monitor the therapy.

Graphical representations of each electrode orientation are indicated by icons to identify what measured data corresponds to what area of the patient. Icon 154 represents the left needle site, icon 156 represents the right needle site, and icon 158 represents the urethra. Each needle site shows an orientation of each needle with respect to the tissue. Icons 154, 156 and 158 also indicate which channel is being used to ablate tissue. Each icon may be represented by a different color to further distinguish the icon. In other embodiments, words may be used instead of graphical icons.

If the user desires to return to the main menu, exit box 160 may be pressed to exit the therapy screen and return to the main menu. In this embodiment, exit box 160 is the only touch spot on the screen available to the user. Therapy is begun by pressing a button or handle on the connected ablation device. Exit box 160 is a multifunction button. For example, once therapy is started, exit box 160 may change to a stop icon. In other states of PTD 14, exit box 160 may change to other icons as well. Other embodiments may allow further control of the therapy from the touch screen.

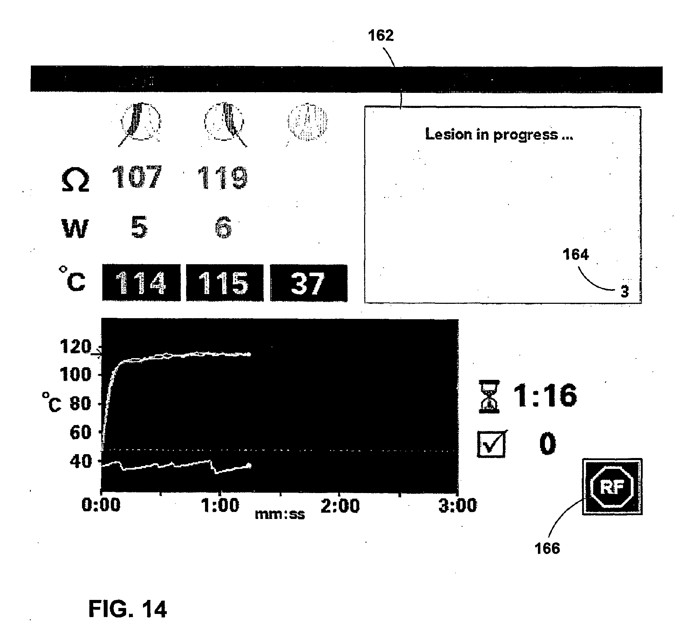

FIG. 14 is an exemplary screen shot of the delivery screen when ablation therapy is being delivered. Message box 162 delivers a message to the user that a lesion is in progress. This message corresponds to the user manual, as indicated by indicator 164. Values for tissue resistance, power, and temperature are displayed in their respective areas. The graph also shows temperature in their appropriate areas. The colors of the temperatures plotted in the graph correspond to the color of each tissue location.

As shown by the timer, remaining time for therapy is counting down. Upon the end of the timer, the system may provide an audible indication of elapsed time, provide a visual cue to cease therapy, or cease therapy delivery automatically. If the physician desires to prematurely end therapy, they physician may press stop box 166. This function may be an appropriate safety measure for dysfunctional therapy or an adverse patient reaction.

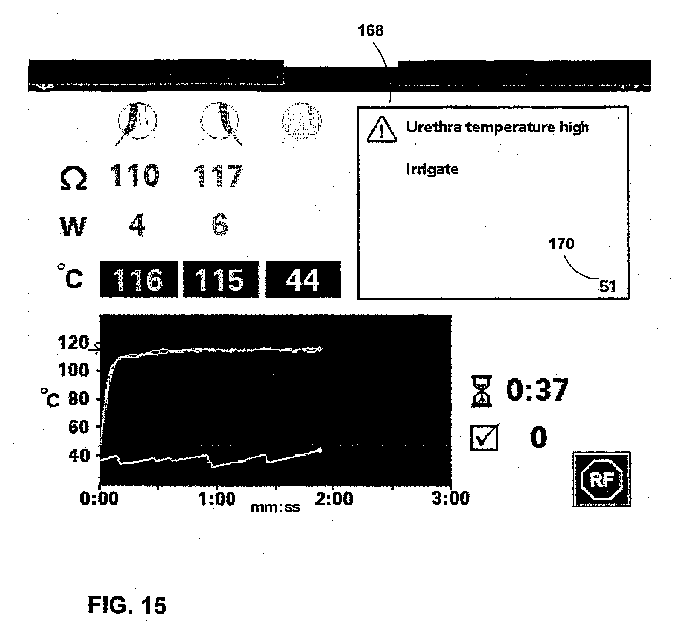

FIG. 15 is an exemplary screen shot of the delivery screen and a temperature warning message during therapy. As indicated by the graph, the temperature of the urethra is reaching an unsafe threshold. Caution message 168 is delivered to advise the physician to irrigate the urethra with fluid to cool the tissue. Indicator 170 corresponds to an index 51 for a user to find further information related to the message.

In some embodiments, the system may automatically shut down therapy if safe temperature levels are breached. In this case, if the urethra was not successfully irrigated with fluid, the therapy may be discontinued automatically or by the physician.

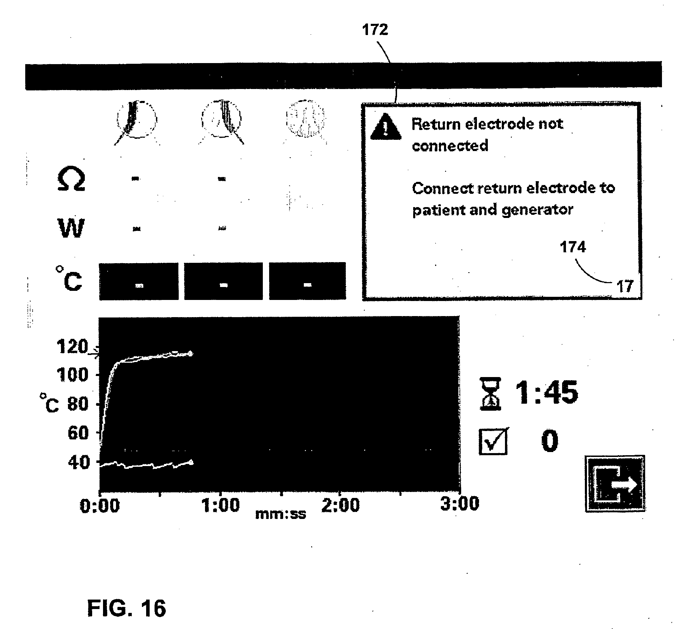

FIG. 16 is an exemplary screen shot of the delivery screen displaying an error message when the therapy is terminated due to the return electrode malfunction. The return electrode allows RF energy to flow from the needle to the return electrode, sometime located on the lower back of the patient. If this return electrode is malfunctioning or removed, the patient could be injured. Warning box 172 informs the user that the return electrode is not connected to the system appropriately. Indicator 174 corresponds to a message index that may be used by a user to find more information related to the problem or message.

In this exemplary embodiment, therapy has been suspended until the return electrode is replaced. Once it is, therapy may resume as normal. In some embodiments, a malfunction of the system may force therapy shut down. If this occurs, the therapy would need to be restarted after the system is operational again.

Warnings such as the one displayed in FIG. 12 may not be the only warning issued by PTD 14. Other warnings may be delivered as well, such as dysfunctional needles, improper impedances and high power output. Each warning may be accompanied by a suggestion for correcting the problem.

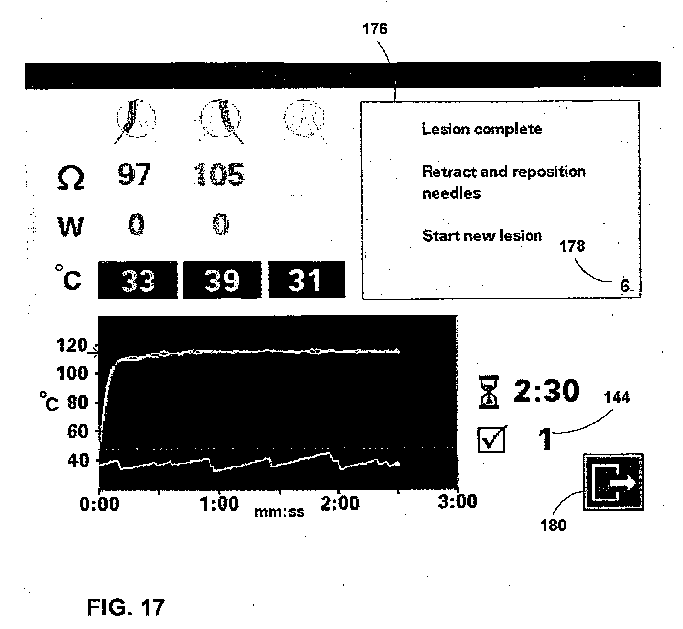

FIG. 17 is an exemplary screen shot of the delivery screen when the therapy is completed. Message box 176 indicates that the lesion created by ablation therapy is complete. Additional information is provided on how to proceed. Indicator 178 shows the user where to find for information regarding the message in the user manual.

Therapy was completed at a site in this screen shot; therefore, one lesion was created. This lesion number is indicated by check box 144. As more lesions are created by the therapy, the number displayed by check box 144 will increase appropriately. Since each patient is different, the number of lesions required to effectively treat a patient may vary from one to many more than one. If no more lesions are required by the user, the user may exit to the menu by pressing exit box 160.

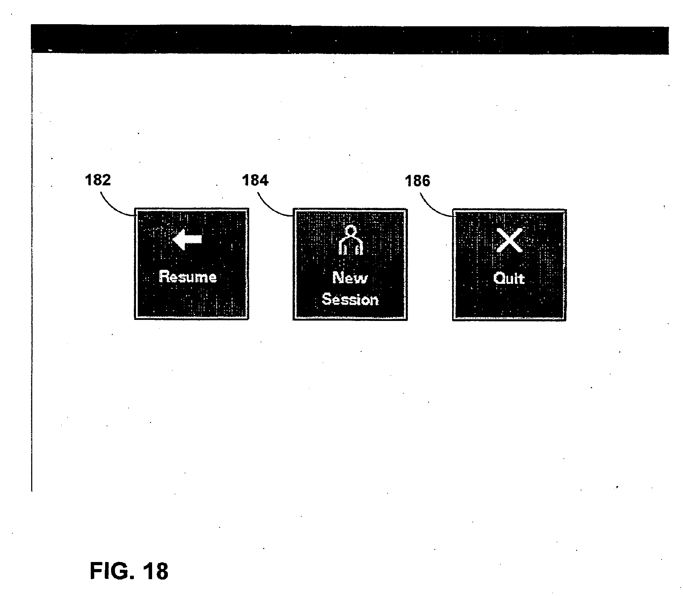

FIG. 18 is an exemplary screen shot of the post session menu. The user may be presented with a variety of choices. By pressing resume box 182, the user may re-enter the therapy screen that the user just exited from. In this case, the user would be free to then create more lesions. If a new session is required, the user may press new session box 184. This option may be used to treat another patient or provide therapy to another location. By pressing quit box 186, the user may return to the main menu to select a new therapy or turn off PTD 14.

In some embodiments, more options may be available for the user. This screen of FIG. 14 may contain additional features which could be modified to the user's preferences. For example, the user may decide to change the color scheme of the indicators, modify the volume, or request different information to be displayed during therapy.

While the screen shots provided in FIGS. 12 though 18 show one type of display for use with PTD 14, many other display formats may be used. These formats may include more or less user modifications, different sized indicators, different colors, pop-up messages, or any other format for displaying the described information pertinent to this RF ablation therapy or any other therapy described herein.

Various embodiments of the described invention may include processors that are realized by microprocessors, Application-Specific Integrated Circuits (ASIC), Field-Programmable Gate Arrays (FPGA), or other equivalent integrated logic circuitry. The processor may also utilize several different types of storage methods to hold computer-readable instructions for the device operation and data storage. These memory and storage media types may include a type of hard disk, random access memory (RAM), or flash memory, e.g. CompactFlash or SmartMedia.

The preceding specific embodiments are illustrative of the practice of the invention. It is to be understood, therefore, that other expedients known to those skilled in the art or disclosed herein may be employed without departing from the scope of the invention as defined by the appended claims.

Many embodiments of the invention have been described. Various modifications may be made without departing from the scope of the claims.

## Claims

1. A portable system, the system comprising:
a device housing (19);
a processor (68) located within the device housing that controls tissue ablation therapy; a visual operation indicator; and
a touch screen user interface (64) controlled by the processor; **characterised by**
a screen housing (26) that accepts the user interface, wherein the screen housing is attached to the device housing via a hinge (36A, 36B); and
wherein
the visual operation indicator (28) is disposed on an exterior surface of the screen housing, wherein the visual operation indicator is a three-sided light bar located at the top of said screen housing comprising a plurality of lights (56, 58, 60) and a cover (35) that encloses the lights.

2. The system of claim 1, wherein the hinge (36A, 36B) allows the screen housing (26) to rotate about a longitudinal axis of the hinge.

3. The system of claim 1, wherein the screen housing (26) protects the user interface when the screen housing is in a closed configuration.

4. The system of claim 1, further comprising a latch that secures the screen housing (26) against the device housing when the screen housing is in a closed configuration.

5. The system of claim 1, wherein the user interface (64) is viewable to a user when the screen housing is in an open configuration.

6. The system of claim 1, wherein the screen housing (26) comprises at least one of magnesium alloy, aluminum alloy, polycarbonate, polypropylene, polyurethane, polyethylene, and polystyrene.

7. The system of claim 1, wherein the touch screen user interface (64) comprises a sensing system that enables a user to control operation of the portable device by touching graphically defined locations on the touch screen user interface.

8. The system of claim 7, wherein the sensing system is one of a resistive system and a surface acoustic wave system.

9. The system of claim 1, wherein the visual operation indicator fastens two or more pieces of the screen housing.

10. The system of claim 1, wherein the screen housing comprises:
at least one of a video output, an IEEE 1394 port, a universal serial bus port , and a wireless communication antenna; and
a speaker that produces audible sounds to a user.

11. The system of claim 1, wherein the device housing comprises a recessed area that holds device instructions, and wherein the screen housing covers the recessed area when the screen housing is in a closed configuration.

12. The system of claim 1, further comprising:
a connector board port (99);
a connector board (46) that couples to the connector board port;
a signal generator (76) that generates radio frequency energy for the tissue ablation therapy; and
a fluid pump (78) that delivers fluid to a patient during a therapy.

13. A device comprising:
a touch screen user interface (64) controlled by a processor, wherein the touch screen user interface determines tissue ablation therapy; and a visual operation indicator (28); **characterised by**
a screen housing (26) that surrounds the user interface;
a hinge (36A, 36B) attached to the screen housing, wherein the screen housing rotates about a longitudinal axis of the hinge; and wherein
the visual operation indicator (28) is disposed on an exterior surface of the screen housing, wherein:
the visual operation indicator is a three-sided light bar located at the top of said screen housing comprising a plurality of lights (56, 58, 60) and a cover (35) that encloses the lights; and
the screen housing protects the touch screen user interface when the screen housing is in a closed configuration.

14. The device of claim 13, further comprising a latch that secures the screen housing against a device housing when the screen housing is in a closed configuration.

15. The device of claim 13, wherein the screen housing comprises at least one of magnesium alloy, aluminum alloy, polycarbonate, polypropylene, polyurethane, polyethylene, and polystyrene.

16. The device of claim 13, wherein the touch screen user interface comprises a sensing system that enables a user to control operation of the device by touching graphically defined locations on the touch screen user interface.

17. The device of claim 13, wherein the visual operation indicator fastens two or more pieces of the screen housing.

18. The device of claim 13, wherein the screen housing comprises:
at least one of a video output, an IEEE 1394 port, a universal serial bus port, and a wireless communication antenna; and
a speaker that produces audible sounds to a user.

## Patentansprüche

1. Portables System, wobei das System aufweist:
ein Vorrichtungsgehäuse (19);
einen in dem Vorrichtungsgehäuse angeordneten Prozessor (68), der eine Gewebeablationstherapie steuert;
eine visuelle Betriebsanzeige; und
ein Touchscreen-Benutzerinterface (64), das durch den Prozessor gesteuert wird; **gekennzeichnet durch**
ein Bildschirmgehäuse (26), das das Benutzerinterface aufnimmt, bzw. akzeptiert, wobei das Bildschirmgehäuse an das Vorrichtungsgehäuse über ein Gelenk bzw. Scharnier (36A, 36B) angebracht ist; und wobei
die visuelle Betriebsanzeige (28) auf einer äußeren Oberfläche des Bildschirmgehäuses angebracht ist, wobei die visuelle Betriebsanzeige ein dreiseitiger Lichtstab bzw. eine dreiseitige Lichtleiste mit einer Anzahl von Lichtern bzw. Lampen (56, 58, 60) und einer die Lampen einschließenden Abdeckung (35) ist, der bzw. die auf der Oberseite des Bildschirmgehäuses angeordnet ist.

2. System nach Anspruch 1, bei dem das Gelenk (36A, 36B) es dem Bildschirmgehäuse (26) ermöglicht, sich um eine Längsachse des Gelenks zu drehen.

3. System nach Anspruch 1, bei dem das Bildschirmgehäuse (26) das Benutzerinterface schützt, wenn sich das Bildschirmgehäuse in einer geschlossenen Konfiguration befindet.

4. System nach Anspruch 1, das ferner einen Riegel aufweist, der das Bildschirmgehäuse (26) gegen das bzw. an dem Vorrichtungsgehäuse befestigt, wenn sich das Bildschirmgehäuse in einer geschlossenen Konfiguration befindet.

5. System nach Anspruch 1, bei dem das Benutzerinterface (64) für einen Benutzer sichtbar bzw. betrachtbar ist, wenn sich das Bildschirmgehäuse in einer offenen Konfiguration befindet.

6. System nach Anspruch 1, bei dem das Bildschirmgehäuse (26) wenigstens eines der folgenden aufweist:
Magnesiumlegierung, Aluminiumlegierung, Polycarbonat, Polypropylen, Polyurethan, Polyethylen und Polystyrol.

7. System nach Anspruch 1, bei dem das Touchscreen-Benutzerinterface (64) ein Erfassungssystem aufweist, das es einem Benutzer ermöglicht, einen Betrieb der portablen Vorrichtung durch Berühren graphisch definierter Orte auf dem Touchscreen-Benutzerinterface zu steuern.

8. System nach Anspruch 7, bei dem das Erfassungssystem ein resistives System oder ein Oberflächen-Akustikwellensystem ist.

9. System nach Anspruch 1, bei dem die visuelle Betriebsanzeige zwei oder mehr Teile des Bildschirmgehäuses befestigt.

10. System nach Anspruch 1, bei dem das Bildschirmgehäuse aufweist:
einen Videoausgang, einen IEEE 1394-Ausgang, einen seriellen universellen Bus-Ausgang bzw. USB-Ausgang und/oder eine Drahtlos-Kommunikations-Antenne; und
einen Lautsprecher, der hörbare Töne für einen Benutzer produziert.

11. System nach Anspruch 1, bei dem das Vorrichtungsgehäuse eine ausgesparte bzw. vertiefte Region aufweist, die Vorrichtungsanweisungen beinhaltet bzw. hält, und bei dem das Bildschirmgehäuse die ausgesparte Region abdeckt, wenn sich das Bildschirmgehäuse in einer geschlossenen Konfiguration befindet.

12. System nach Anspruch 1, das ferner aufweist:
einen Verbindungsboardanschluss (99);
ein Verbindungsboard (46) das mit dem Verbindungsboardanschluss koppelt bzw. verbunden ist;
einen Signalgenerator (76) der Hochfrequenzenergie für die Gewebeablationstherapie erzeugt; und
eine Fluidpumpe (78), die ein Fluid an einen Patienten während einer Therapie bereitstellt.

13. Vorrichtung, die aufweist:
ein durch einen Prozessor gesteuertes Touchscreen-Benutzerinterface (64), wobei das Touchscreen-Benutzerinterface eine Gewebeablationstherapie bestimmt; und
eine visuelle Betriebsanzeige (28); **gekennzeichnet durch**
ein Bildschirmgehäuse (26), das das Benutzerinterface umgibt;
ein Gelenk bzw. Scharnier (36A, 36B), das an das Bildschirmgehäuse angebracht ist, wobei sich das Bildschirmgehäuse um eine Längsachse des Scharniers dreht, und wobei
die visuelle Betriebsanzeige (28) auf einer äußeren Oberfläche des Bildschirmgehäuses angeordnet ist, wobei:
die visuelle Betriebsanzeige ein dreiseitiger Lichtstab bzw. eine dreiseitige Lichtleiste mit einer Anzahl von Lichtern bzw. Lampen (56, 58, 60) und einer die Lampen einschließenden Abdeckung (35) ist, der bzw. die auf der Oberseite des Bildschirmgehäuses angeordnet ist; und
wobei das Bildschirmgehäuse das Touchscreen-Benutzerinterface schützt, wenn sich das Bildschirmgehäuse in einer geschlossenen Konfiguration befindet.

14. Vorrichtung nach Anspruch 13, ferner mit einem Riegel, der das Bildschirmgehäuse gegen ein bzw. an einem Vorrichtungsgehäuse befestigt, wenn sich das Bildschirmgehäuse in einer geschlossenen Konfiguration befindet.

15. Vorrichtung nach Anspruch 13, bei dem das Bildschirmgehäuse wenigstens eines der folgenden aufweist:
Magnesiumlegierung, Aluminiumlegierung, Polycarbonat, Polypropylen, Polyurethan, Polyethylen und Polystyrol.

16. Vorrichtung nach Anspruch 13, bei der das Touchscreen-Benutzerinterface ein Erfassungssystem aufweist, das es einem Benutzer ermöglicht, einen Betrieb der Vorrichtung durch Berühren graphisch definierter Orte auf dem Touchscreen-Benutzerinterface zu steuern.

17. Vorrichtung nach Anspruch 13, bei der die visuelle Betriebsanzeige zwei oder mehr Teile des Bildschirmgehäuses befestigt.

18. Vorrichtung nach Anspruch 13, bei der das Bildschirmgehäuse aufweist:
einen Videoausgang, einen IEEE 1394-Anschluss, einen universellen seriellen Bus-Anschluss bzw. USB-Anschluss und/oder eine Drahtlos-Kommunikationsantenne; und
einen Lautsprecher, der für bzw. an einen Benutzer hörbare Töne bereitstellt.

## Revendications

1. Système portable, le système comportant :
un boîtier de dispositif (19) ;
un processeur (68) localisé dans le boîtier de dispositif qui commande une thérapie d'ablation de tissu ;
un indicateur d'opération visuel ; et
une interface utilisateur à écran tactile (64) commandée par le processeur ; **caractérisé par**
un boîtier d'écran (26) qui accepte ou reçoit l'interface utilisateur, le boîtier d'écran étant fixé au boîtier de dispositif via une articulation (36A, 36B) ; et dans lequel l'indicateur d'opération visuel (28) est disposé sur une surface extérieure du boîtier d'écran, l'indicateur d'opération visuel est une barre lumineuse à trois côtés positionnée au-dessus dudit boîtier d'écran comportant une pluralité de lampes ou lumières (56, 58, 60) et un couvercle (35) qui enferme les lampes.

2. Système selon la revendication 1, dans lequel l'articulation (36A, 36B) permet au boîtier d'écran (26) de tourner autour d'un axe longitudinal de l'articulation.

3. Système selon la revendication 1, dans lequel le boîtier d'écran (26) protège l'interface utilisateur lorsque le boîtier d'écran est dans une configuration fermée.

4. Système selon la revendication 1, comportant en outre un verrou qui fixe le boîtier d'écran (26) vis-à-vis du boîtier de dispositif lorsque le boîtier d'écran est dans une configuration fermée.

5. Système selon la revendication 1, dans lequel l'interface utilisateur (64) est visualisable ou visible pour un utilisateur lorsque le boîtier d'écran est dans une configuration ouverte.

6. Système selon la revendication 1, dans lequel le boîtier d'écran (26) comporte au moins l'un parmi un alliage de magnésium, un alliage d'aluminium, du polycarbonate, du polypropylène, du polyuréthane, du polyéthylène, et du polystyrène.

7. Système selon la revendication 1, dans lequel l'interface utilisateur à écran tactile (64) comporte un système de détection qui permet à un utilisateur de commander le fonctionnement du dispositif portable en touchant des emplacements définis graphiquement sur l'interface utilisateur à écran tactile.

8. Système selon la revendication 7, dans lequel le système de détection est l'un parmi un système résistif et un système à ondes acoustiques de surface.

9. Système selon la revendication 1, dans lequel l'indicateur d'opération visuel attache deux ou plus de deux éléments du boîtier d'écran.

10. Système selon la revendication 1, dans lequel le boitier d'écran comporte :
au moins l'un parmi une sortie vidéo, un port IEEE 1394, un port de bus série universel (Universal Serial Bus) et une antenne de communication sans fil ; et
un haut-parleur qui produit des sons audibles pour un utilisateur.

11. Système selon la revendication 1, dans lequel le boitier de dispositif comporte une zone en retrait qui contient des instructions du dispositif, et dans lequel le boîtier d'écran recouvre la zone en retrait lorsque le boîtier d'écran est dans une configuration fermée.

12. Système selon la revendication 1, comportant en outre :
un port de carte de connexion (99) ;
une carte de connexion (46) qui est couplée au port de carte de connexion ;
un générateur de signal (76) qui génère une énergie de fréquence radio pour la thérapie d'ablation de tissu ; et
une pompe à fluide (78) qui délivre un fluide à un patient pendant une thérapie.

13. Dispositif comportant :
une interface utilisateur à écran tactile (64) commandée par un processeur, dans lequel l'interface utilisateur à écran tactile détermine une thérapie d'ablation de tissu ; et un indicateur d'opération visuel (28) ; **caractérisé par**
un boîtier d'écran (26) qui entoure l'interface utilisateur ;
une articulation (36A, 36B) fixée au boîtier d'écran, le boîtier d'écran tournant autour d'un axe longitudinal de l'articulation ; et dans lequel
l'indicateur d'opération visuel (28) est disposé sur une surface extérieure du boîtier d'écran, dans lequel :
l'indicateur d'opération visuel est une barre lumineuse à trois côtés positionnée au-dessus dudit boîtier d'écran comportant une pluralité de lampes ou lumières (56, 58, 60) et un couvercle (35) qui enferme les lampes ; et
le boîtier d'écran protège l'interface utilisateur à écran tactile lorsque le boîtier d'écran est dans une configuration fermée.

14. Dispositif selon la revendication 13, comportant en outre un verrou qui fixe le boîtier d'écran vis-à-vis un boîtier de dispositif lorsque le boîtier d'écran est dans une configuration fermée.

15. Dispositif selon la revendication 13, dans lequel le boîtier d'écran comporte au moins l'un parmi un alliage de magnésium, un alliage d'aluminium, du polycarbonate, du polypropylène, du polyuréthane, du polyéthylène, et du polystyrène.

16. Dispositif selon la revendication 13, dans lequel l'interface utilisateur à écran tactile comporte un système de détection qui permet à un utilisateur de commander le fonctionnement du dispositif en touchant des emplacements définis graphiquement sur l'interface utilisateur à écran tactile.

17. Dispositif selon la revendication 13, dans lequel l'indicateur d'opération visuel attache deux ou plus de deux éléments du boîtier d'écran.

18. Dispositif selon la revendication 13, dans lequel le boitier d'écran comporte :
au moins l'un parmi une sortie vidéo, un port IEEE 1394, un port de bus série universel (Universal Serial Bus) et une antenne de communication sans fil ; et
un haut-parleur qui produit des sons audibles pour un utilisateur.
